# EUROPEAN PATENT SPECIFICATION

(11) **EP 2 424 596 B1**
(45) Date of publication and mention of the grant of the patent: **28.02.2024**
(21) Application number: 10770407.4
(22) Date of filing: 30.04.2010
(51) Int. Cl.: A61M 5/24, A61J 1/06, A61M 5/28, A61B 17/70, A61B 17/34, A61B 17/00, A61M 31/00, A61M 35/00, B65D 81/32, A61M 5/00

(54) **APPLICATOR SYSTEM FOR THE DELIVERY OF SURGICAL SEALANTS**
APPLIKATORSYSTEM ZUR VERABREICHUNG CHIRURGISCHER DICHTUNGSMITTEL
SYSTÈME APPLICATEUR POUR LA DISTRIBUTION D'AGENTS DE SCELLEMENT CHIRURGICAUX

(30) Priority: 30.04.2009 US 174150 P
(43) Date of publication of application: 07.03.2012
(73) Proprietor: Hyperbranch Medical Technology, Inc., Durham, NC 27713-4410 (US)
(72) Inventor: D'ALESSIO, Keith, R., Cary NC 27511 (US); CARNAHAN, Michael, A., Durham NC 27713 (US); BUTLIN, Jared, D.G., Durham NC 27713 (US)
(74) Representative: HGF
(86) International application number: PCT/US2010/033134
(87) International publication number: WO 2010/127222

(56) References cited:
- WO-A1-92/10225
- WO-A2-2009/015124
- US-A- 4 572 210
- US-A1- 2001 047 187
- US-A1- 2004 138 611
- US-A1- 2008 188 828
- US-B1- 6 818 018
- US-B2- 6 949 114

## Description

### RELATED APPLICATION

This application claims the benefit of priority to United States Provisional Patent Application serial number 61/174,150, filed April 30 2009.

### BACKGROUND

Small spinal dural defects (e.g., on the order of 1-3 mm) are sometimes an inadvertent consequence of a surgical procedure to remove or repair other structures within the spine. A physician confronted with such a defect is unlikely to reach for a complicated applicator system, which carries a large payload of sealant formulation, but would instead prefer a simple, direct placement of a much smaller amount of a sealant formulation. Applicator systems that allow for direct placement of small amounts of sealant formulations, and methods for their use, are described

A syringe according to the preamble of claim 1 is known from document US2001047187.

### SUMMARY

The present invention relates to a syringe as defined in claim 1, comprising: a syringe body having a sidewall, and an opening at each of its proximal and distal ends; a floating hydrophobic septum contacting the sidewall of the syringe body and translatable from a position near the proximal end of the syringe body to a position at the distal end of the syringe body, thereby creating a top compartment, bounded by the proximal end of the syringe and the hydrophobic septum, and a bottom compartment, bounded by the distal end of the syringe and the hydrophobic septum, wherein the hydrophobic septum comprises a hydrophobic porous filter; a liquid in the top compartment, the liquid being an aqueous solution which comprises phosphates or borates; a polymerizable component in the bottom compartment; and a plunger having a shaft, a distal end, a proximal end, and a plunger head provided at the distal end; the plunger inserted into the syringe body plunger head first through the proximal opening of the syringe body; the plunger head configured and adapted to form a seal when the plunger head engages the sidewall of the syringe body, wherein the plunger is axially translatable between a position where the plunger head is at the proximal end of the syringe body and a position wherein the plunger head is flush with the hydrophobic septum, wherein the hydrophobic septum repels the liquid such that the liquid does not pass through the hydrophobic septum absent pressure applied by the plunger.

Preferred embodiments are defined in the dependent claims.

More detailed and specific aspects of the present invention will be understood by reference to the following figures and detailed description which illustrate by way of example a few of the forms of the inventions.

### BRIEF DESCRIPTION OF THE FIGURES

**Figure 1** depicts an applicator system a syringe body, a plunger, an adaptor, a bladder and a straight tip.
**Figure 2** depicts another applicator system comprising a syringe body, a plunger, an adaptor, a bladder and a bent tip.
**Figure 3** depicts a syringe body, a plunger, a hydrophobic filter, and a hydrophilic filter containing a polymerizable component, such as PEI, adsorbed thereto.
**Figure 4** depicts a syringe body, a plunger and a floating plunger head with a check valve or the like.
**Figure 5** depicts the application of a hydrogel to spinal dura.
**Figure 6** is a graph showing the average maximum sustained cerebrospinal fluid pressure achieved during intra-operative pressure testing between control (black; sutures only) and treated (white; sutures and hydrogel) groups.
**Figure 7** depicts an embodiment of a septum and an applicator which uses the septum and a plastic ampoule (rather than a syringe body).

### DETAILED DESCRIPTION

There is a need to develop improved dispensers that facilitate the complete mixing of solids and/or liquids inside the dispenser while maintaining the sterility of the mixture. In addition, there is need for a system for dispensers that allow two or more components which are to be mixed to be kept separate until just prior to use. Further, it would be advantageous if the dispensers could also act as applicators, thereby facilitating the application of the mixture. The present invention addresses these needs and others.

The present disclosure relates to an applicator system that may be used to house multiple components (e.g., components of a polymerizable hydrogel, such as solids and liquids), facilitating the mixing of the components inside the applicator, and further facilitating the application of the mixture. Another aspect of the disclosure relates to an applicator system that may be used to house multiple liquids and multiple solids (*e*.*g*., components of a polymerizable hydrogel), facilitating the mixing of the solids and liquids inside the applicator, and further facilitating the application of the mixture. Another aspect of the disclosure relates to an applicator system that may be used to house one liquid and one solid (*e*.*g*., components of a polymerizable hydrogel), facilitating the mixing of the solid and liquid inside the applicator, and further facilitating the application of the mixture.

While the invention will often be described herein as facilitating the formation and effective delivery of a polymerizable hydrogel formulation to a patient, this presentation is not intended in any way to limit the scope of the invention to such an application.

In certain embodiments not part of the present invention, applicators can be used to prepare and apply a hydrogel formulation. The applicators can be used to prepare and apply a hydrogel formulation to dura matter (such as for use in the sealing of spinal column dura mater following spinal surgery). The hydrogel formulation is delivered in liquid form and quickly polymerizes into a soft hydrogel. In certain embodiments, the hydrogel formulation comprises three basic components: a cross linker (such as PEI); an activated polymer (such as activated PEG); and a buffer solution.

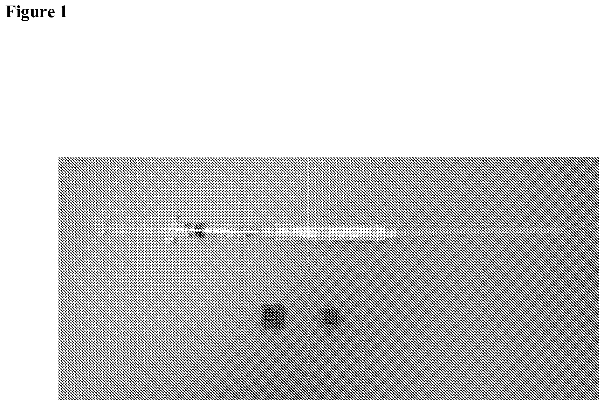

In certain embodiments not part of the present invention, the applicators comprise a syringe which is used for the storage of a liquid portion of a formulation; a bladder which is used for the storage of the solid portion, and used for the mixing of the liquid and solid portions; and a tip or cap on the proximal end of the bladder or flexible tube. The use of a syringe for the storage of the liquid portion of the formulation offers the advantage of at least two modes of operation for a given applicator. The applicator device is assembled at the time of use and the sealant formulation is reconstituted by using the syringe to force the liquid portion of the formulation (e.g., PEI in buffer) into the bladder or flexible tubing which contains the solid portion of the formulation (e.g., an activated PEG powder filled bladder or flexible tubing). Shaking the applicator mixes the formulation components and readies the device for delivery. The mixed formulation can then be delivered dropwise and precisely by manually compressing the bladder or flexible tubing. Alternatively, such an applicator could also then be used to deliver a larger bolus of formulation by withdrawing the syringe plunger and then advancing the syringe forward to expel the formulation. An example of an applicator can be seen in Figure 1.

A check valve is added to the proximal end of the luer lock to tubing adapter of the device shown in Figure 1. This addition allows the syringe to be removed after the reconstitution phase just prior to application of the formulation. In certain embodiments the check valve can be configured to allow for air to only pass into, not out of, the bladder.

In certain embodiments, a PEI-containing solution is held within the syringe. Although such embodiments are operative, at a 10% solids loading the hydrogel formed using a sterilized PEI containing buffer solution suffered from a higher than desired percent swelling after placement in phosphate buffered saline (PBS) at 37° C for 24 hours.

The applicators comprise a polymerizable component adsorbed onto a dissolution aid scaffold (e.g., a frit or a porous filter). In certain embodiments, the polymerizable component is a viscous liquid (e.g., PEI). In certain embodiments, the dissolution aid scaffold is located between the bladder and the syringe, for example, within the luer lock tubing adaptor. One advantage to using a polymerizable component adsorbed onto a dissolution aid scaffold is that it can be kept separate from the other components during radiation sterilization. For example, to form a hydrogel with such embodiments, at the time of use a buffer solution is forced from the syringe through, for example, a PEI-containing frit and into a PEG-loaded bladder. PEI/PEG hydrogels prepared by such embodiments showed only a low minimal level of swelling after soaking in 37° C PBS for 24 hours.

While the system described above is workable, it was observed that there was a tendency for the buffer solution to pass through the PEI-containing frit and prematurely form hydrogel globules within the PEG filled flexible tubing member. This in turn caused plugging of the delivery cannula and/or filter within the device. Most likely this was due to a predominance of the PEI coming off of the frit early in the buffer solution flow through, thus causing a high to low concentration gradient of PEI to mix with the activated PEG within the applicator.

Therefore, an alternative means of introducing the buffer solution to PEI after sterilization (i.e., embodiments which minimized or eliminated the PEI gradient which is formed with a straight through flow of buffer through a PEI frit) were devised. One such embodiment utilizes a normal syringe to which a floating hydrophilic filter media (e.g., a Filtrona Filter) has been inserted into the distal third end of the interior aspect of the syringe. PEI solution is deposited onto the hydrophilic filter media and can optionally be vacuum dried. A septum is then inserted into the interior aspect of the syringe somewhat proximal to the hydrophilic filter. The septum is hydrophobic in nature and therefore repels aqueous solutions. Lastly a buffer solution is added to the most proximal end of the syringe and sealed with a normal syringe plunger. A cap was also added to the most distal end of the syringe.

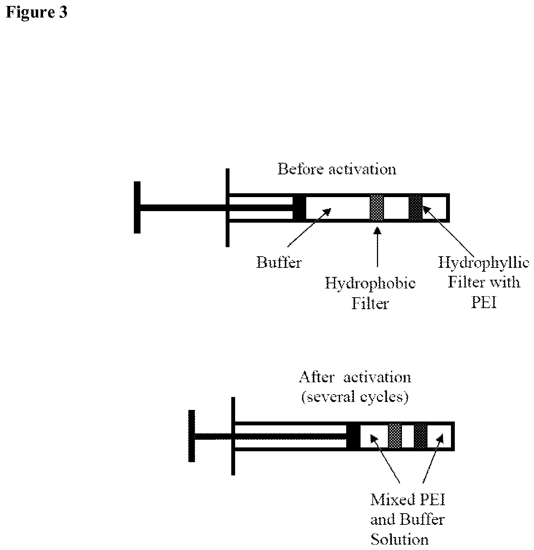

Importantly, the hydrophobic nature of the septum prevents the aqueous based buffer solution from passing absent pressurization, thus effectively separating the buffer from the PEI at the time of sterilization and prior to use. At time of use, the syringe plunger is manually advanced which drives the buffer solution through the septum and into the PEI-containing hydrophilic filter. If desired several cycles of manually depressing the plunger and releasing the plunger can be used to thoroughly mix the PEI into solution. Only then is the cap of the syringe removed and the syringe attached to the PEG filled bladder. The plunger can then be depressed and the reconstituted PEI solution expelled from the syringe until floating septum and floating hydrophilic filter media have been pushed to the distal end of the syringe. A drawing of such a device is shown below in Figure 3.

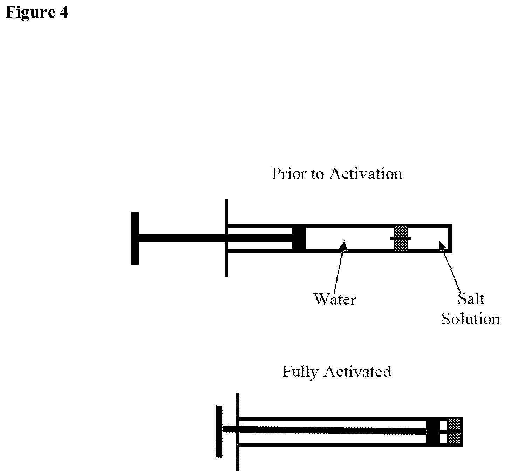

In addition, a similar action can be performed by different arrangements. For example, certain arrangements of the applicator comprise a non-porous floating plunger which is placed into the central portion of the interior portion of a syringe. This floating plunger has no plunger handle but will slide within the interior aspect of the syringe when pressurized. The floating plunger also incorporates a miniature check valve which allows fluid to flow in the distal direction only. The crack pressure of the valve is sized such that the floating plunger will slide forward first before the check valve cracks and allows fluid flow. In the distal end of the syringe there is a small volume of solution with all of the salt components required for a PEG/PEI hydrogel with a desired set time to form under normal conditions. In certain embodiments not part of the present invention, the proximal end of the syringe is filled with an amount of water such that once mixed the water and the salt component solution will make the correct reconstituted PEI buffer solution. In such embodiments, the PEI can be introduced via a dissolution aid scaffold (such as an impregnated frit) which can be housed within the tubing to luer lock adapter, similar to that described above. Such two-liquid syringes are sterilized separate from the PEI frit/PEG filled flexible tubing member, thus avoiding decomposition of the PEI during sterilization. At time of use, the device is assembled and the plunger is manually depressed. This action causes the water solution to be pressurized. The pressurized water solution bears against the proximal surface of the floating plunger and forces it forward (without opening the check valve). This action in turn expels the concentrated salt solution through the PEI frit and into the PEG filled flexible tubing member. Since the solution is high in salt content, the PEG dissolves but does not gel even though there is a high to low gradient of PEI as in the prior frit embodiment. Continued manual depression of the plunger causes a forward motion of the floating plunger until it hits the most distal end of the interior aspect of the syringe. At this point further depression of the plunger causes a rise in pressure of the water solution until the crack pressure of the check valve is reached whereupon the valve opens and allows the water to flow into the PEG filled flexible tubing member. The addition of the water results in a solution with the correct pH for polymerization of the hydrogel to occur. A picture of the syringe portion of this embodiment is shown in Figure 4. In other embodiments, a similar two liquid syringe can be configured with a bypass built into the outer part of the syringe instead of a check valve.

In related embodiments, instead of a salt solution and a water solution being combined to form a buffer solution, the buffer components (e.g., phosphates and borates) can likewise be separated in the apparatus and combined only once the polymerizable component has been reconstituted.

In certain embodiments, the applicators of the invention comprise a thin, rigid polymeric tip. Minimally invasive spinal surgery is typically performed through a small incision and a small tube approximately 18 to 22 mm in inside diameter and 40 to 80 cm in length is inserted into the incision and becomes the extent of the operating field. The physician looks through a microscope positioned to have a clear view down the interior aspect of the tube. An applicator comprising a thin rigid tube would allow a user to place discrete drops of formulation where intended while looking through the microscope.

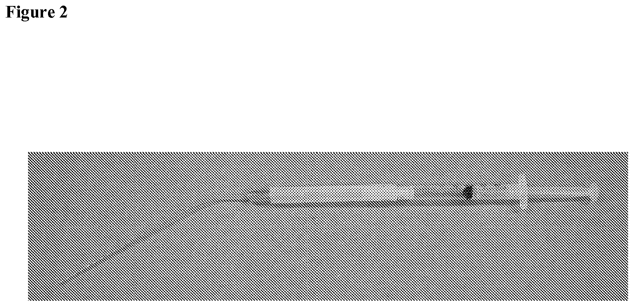

In certain embodiments, in order to avoid the hand of the user blocking the view of the surgical field through the microscope, certain applicators comprise a bent cannula tip or a bayonet-style cannula tip. An example of such a device is pictured in Figure 2. A bayonet style instrument has two opposite angle bends, which together bring the handle of the device in a parallel but offset orientation to the long axis of the distal end of the instrument. This allows the instrument handle to be parallel to the rest of the instrument but it lies outside of the line of sight through, for example, a Minimally Invasive Surgery (MIS) port.

In certain embodiments, the applicators comprise a cannula tip as the distal most portion of the device. However, it should be understood that in other embodiments, the device can be easily be configured to have a brush applicator tip, a duck-bill tip, a silicon tube or any of a myriad of other style tips as might best serve the need or application.

In order to avoid leaking from the end of the applicator immediately after pressure has ceased to be applied to the plunger(s), activated PEG with a higher than average bulk density might be used. Not intending to be bound by any one theory, it is hypothesised that air, both dissolved air and foaminess within the reconstituted material (e.g., activated PEG), could be compressed during the application phase and then result in expression of some small amount of reconstituted component after stopping. With regards to activated PEG, it is further known that the current recrytallization method yields a fluffy powder that has a fairly low bulk density of approximately 0.25 g/cc. It was theorized that if fairly dense but small particles of PEG were used that one could reduce the amount of air in the reconstituted PEG and thus reduce the unintended delivery of reconstituted PEG after stopping. Examples of denser PEG materials are described in Example 7 below.

### SELECTED SYRINGES, AMPOULES AND APPLICATORS

The present invention relates to a syringe, comprising:
a syringe body having a sidewall, and an opening at each of its proximal and distal ends;
a floating hydrophobic septum contacting the sidewall of the syringe body and translatable from a position near the proximal end of the syringe body to a position at the distal end of the syringe body, thereby creating a top compartment, bounded by the proximal end of the syringe and the hydrophobic septum, and a bottom compartment, bounded by the distal end of the syringe and the hydrophobic septum, wherein the hydrophobic septum comprises a hydrophobic porous filter;
a liquid in the top compartment, the liquid being an aqueous solution which comprises phosphates or borates;
a polymerizable component in the bottom compartment; and
a plunger having a shaft, a distal end, a proximal end, and a plunger head provided at the distal end; the plunger inserted into the syringe body plunger head first through the proximal opening of the syringe body; the plunger head configured and adapted to form a seal when the plunger head engages the sidewall of the syringe body, wherein the plunger is axially translatable between a position where the plunger head is at the proximal end of the syringe body and a position wherein the plunger head is flush with the hydrophobic septum, wherein the hydrophobic septum repels the liquid such that the liquid does not pass through the hydrophobic septum absent pressure applied by the plunger.

In certain embodiments, the present invention relates to any one of the aforementioned syringes, further comprising a cap, luer lock or frangible seal at the distal end of the syringe body. In certain embodiments, the present invention relates to any one of the aforementioned syringes, further comprising a cap at the distal end of the syringe body.

In certain embodiments, the present invention relates to any one of the aforementioned syringes, wherein the liquid in the top compartment is a buffer.

In certain embodiments, the present invention relates to any one of the aforementioned syringes, wherein the polymerizable component comprises a polyalkyleneimine. In certain embodiments, the present invention relates to any one of the aforementioned syringes, wherein the polymerizable component comprises PEI.

In certain embodiments, the present invention relates to any one of the aforementioned syringes, wherein the bottom compartment further comprises a liquid so that the polymerizable component is about 20% w/w; about 30% w/w; about 40% w/w; about 50% w/w; about 60% w/w; about 70% w/w; about 90% w/w; or about 90% w/w.

In certain embodiments, the present invention relates to any one of the aforementioned syringes, wherein the syringe body is tubular. In certain embodiments, the present invention relates to any one of the aforementioned syringes, wherein the syringe body is plastic.

In certain embodiments, the present invention relates to any one of the aforementioned syringes, wherein the length of the syringe body is between about 25,4mm (1") and about 127mm (5").

In certain embodiments, the present invention relates to any one of the aforementioned syringes, wherein the length of the syringe body is between about 50,8mm (2") and about 101,6mm (4").

In certain embodiments, the present invention relates to any one of the aforementioned syringes, wherein the length of the syringe body is about 76,2mm (3").

In certain embodiments, the present invention relates to any one of the aforementioned syringes, wherein the length of the syringe body is about 50,4mm (2").

In certain embodiments, the present invention relates to any one of the aforementioned syringes, wherein the length of the syringe body is about 25,4mm (1").

In certain embodiments, the present invention relates to any one of the aforementioned syringes, wherein the average outer diameter of the syringe body is between about 2,54mm (0,1") and about 25,4mm (1").

In certain embodiments, the present invention relates to any one of the aforementioned syringes, wherein the average outer diameter of the syringe body is between about 2,54mm (0,1") and about 10,16mm (0,4").

In certain embodiments, the present invention relates to any one of the aforementioned syringes, wherein the average outer diameter of the syringe body is between about 10,16mm (0,4") and about 15,24mm (0,6").

In certain embodiments, the present invention relates to any one of the aforementioned syringes, wherein the average outer diameter of the syringe body is between about 15,24mm (0,6") and about 20,32mm (0,8").

In certain embodiments, the present invention relates to any one of the aforementioned syringes, wherein the average outer diameter of the syringe body is between about 20,32mm (0,8") and about 25,4mm (1"). In certain embodiments, the present invention relates to any one of the aforementioned syringes, wherein the average outer diameter of the syringe body is about 6,604mm (0,26").

In certain embodiments, the present invention relates to any one of the aforementioned syringes, wherein the average outer diameter of the syringe body is about 9,906mm (0,39").

In certain embodiments, the present invention relates to any one of the aforementioned syringes, wherein the average outer diameter of the syringe body is about 13,07mm (0,55").

In certain embodiments, the present invention relates to any one of the aforementioned syringes, wherein the average outer diameter of the syringe body is about 24,638mm (0,97").

The present invention relates to any one of the aforementioned syringes, wherein the hydrophobic septum comprises a hydrophobic porous filter. In certain embodiments, the present invention relates to any one of the aforementioned syringes, wherein the hydrophobic septum is a hydrophobic porous filter. In certain embodiments, the present invention relates to any one of the aforementioned syringes, wherein the hydrophobic porous filter comprises a flat sheet, hollow fiber, or spiral wound membrane. In certain embodiments, the present invention relates to any one of the aforementioned syringes, wherein the hydrophobic septum comprises polyethylene, polypropylene, polytetrafluoroethylene, polyvinylidene fluoride, polysulfone, polyethersulfone, polyvinyl chloride, or combinations thereof.

In certain embodiments, the present invention relates to any one of the aforementioned syringes, wherein the hydrophobic septum is at least 6,35mm (0,25") from the proximal end of the syringe body. In certain embodiments, the present invention relates to any one of the aforementioned syringes, wherein the hydrophobic septum is at least about 12,7mm (0,5") from the proximal end of the syringe body. In certain embodiments, the present invention relates to any one of the aforementioned syringes, wherein the hydrophobic septum is at least about 19,05mm (0,75") from the proximal end of the syringe body.

In certain embodiment, the present invention relates to any one of the aforementioned syringes, wherein the inside of the bottom compartment is coated with the polymerizable component.

In certain embodiments, the present invention relates to any one of the aforementioned syringes, further comprising a dissolution aid scaffold; wherein the polymerizable component is adsorbed onto the dissolution aid scaffold. In certain embodiment, the present invention relates to any one of the aforementioned syringes, wherein the dissolution aid scaffold is a piece of porous material. In certain embodiment, the present invention relates to any one of the aforementioned syringes, wherein the dissolution aid scaffold is a piece of porous material which is configured and adapted so that fluid passing through the bottom compartment and out the distal end of the syringe body passes through the piece of porous material and reconstitutes the polymerizable component adsorbed thereon.

In certain embodiments, the present invention relates to any one of the aforementioned syringes, wherein the dissolution aid scaffold is a piece of plastic or glass. In certain embodiments, the present invention relates to any one of the aforementioned syringes, wherein the dissolution aid scaffold is a piece of polyethylene (PE), polypropylene (PP), high-density polyethylene (HDPE), ultra-high molecular weight polyethylene (UHMWPE), polytetrafluoroethylene (PTFE), nylon, polyvinylidine fluoride (PVDF), polyethersulfone (PES), ethyl vinyl acetate (EVA), or a co-polymer thereof. In certain embodiments, the present invention relates to any one of the aforementioned syringes, wherein the dissolution aid scaffold is a piece of polyester batting or other fibrous filter media material.

In certain embodiments, the present invention relates to any one of the aforementioned syringes, wherein the dissolution aid scaffold is shaped like a disc and has a diameter equal to that of the syringe body.

In certain embodiments, the present invention relates to any one of the aforementioned syringes, wherein the dissolution aid scaffold is at least about 6,35mm (0,25") from the distal end of the syringe body. In certain embodiments, the present invention relates to any one of the aforementioned syringes, wherein the dissolution aid scaffold is at least about 12,7mm (0,5") from the distal end of the syringe body. In certain embodiments, the present invention relates to any one of the aforementioned syringes, wherein the dissolution aid scaffold is at least about 19,05mm (0,75") from the distal end of the syringe body.

In arrangements not part of the present invention, the disclosure relates to any one of the aforementioned syringes, wherein instead of a hydrophobic septum, a floating plunger head is used, wherein the floating plunger head is positioned between the proximal and distal ends of the syringe body, thereby creating a top compartment, bounded by the proximal end of the syringe and the floating plunger head, and a bottom compartment, bounded by the distal end of the syringe and the floating plunger head; wherein the floating plunger head contains a check valve which, when opened, connects the top compartment and the bottom compartment; the floating plunger head is configured and adapted to form a seal with the sidewall of the syringe body; and the floating plunger is axially translatable from a position near the proximal end of the syringe body to a position at the distal end of the syringe body.

In arrangements not part of the present invention, the disclosure relates to any one of the aforementioned syringes, wherein instead of a hydrophobic septum, a floating plunger head is used, wherein the floating plunger head is positioned between the proximal and distal ends of the syringe body, thereby creating a top compartment, bounded by the proximal end of the syringe and the floating plunger head, and a bottom compartment, bounded by the distal end of the syringe and the floating plunger head; wherein the floating plunger head contains a frangible membrane which, when broken, connects the top compartment and the bottom compartment; the floating plunger head is configured and adapted to form a seal with the sidewall of the syringe body; and the floating plunger is axially translatable from a position near the proximal end of the syringe body to a position at the distal end of the syringe body.

Another aspect of the present disclosure not part of the present invention relates to an ampoule, comprising:
a closed proximal end and a frangible seal at its distal end;
a floating hydrophobic septum positioned inside the ampoule so as to create a top compartment, bounded by the closed proximal end of the ampoule and the hydrophobic septum, and a bottom compartment, bounded by the frangible seal at the distal end of the ampoule and the hydrophobic septum;
a liquid in the top compartment; and
a polymerizable component in the bottom compartment.

In certain embodiments, the present invention relates to any one of the aforementioned ampoules, wherein the liquid in the top compartment is a buffer.

In certain arrangements, the present disclosure relates to any one of the aforementioned ampoules, wherein the polymerizable component comprises a polyalkyleneimine. In certain arrangements, the present disclosure relates to any one of the aforementioned ampoules, wherein the polymerizable component comprises PEI.

In certain arrangements, the present disclosure relates to any one of the aforementioned ampoules, wherein the bottom compartment further comprises a liquid so that the polymerizable component is about 20% w/w; about 30% w/w; about 40% w/w; about 50% w/w; about 60% w/w; about 70% w/w; about 90% w/w; or about 90% w/w.

In certain arrangements, the present disclosure relates to any one of the aforementioned ampoules, wherein the ampoule is tubular. In certain arrangements, the present disclosure relates to any one of the aforementioned ampoules, wherein the ampoule is plastic.

In certain arrangements, the present disclosure relates to any one of the aforementioned ampoules, wherein the length of the ampoule is between about 25,4mm (1") and 127mm (5").

In certain arrangements, the present disclosure relates to any one of the aforementioned ampoules, wherein the length of the ampoule is between about 50,8mm (2") and 101,6mm (4"). In certain arrangements, the present disclosure relates to any one of the aforementioned ampoules, wherein the length of the ampoule is about 76,2mm (3"). In certain arrangements, the present disclosure relates to any one of the aforementioned ampoules, wherein the length of the ampoule is about 50,8mm (2"). In certain arrangements, the present disclosure relates to any one of the aforementioned ampoules, wherein the length of the ampoule is about 25,4mm (1").

In certain arrangements, the present disclosure relates to any one of the aforementioned ampoules, wherein the average outer diameter of the ampoule is between about 2,54mm (0.1") and about 25,4mm (1"). In certain arrangements, the present disclosure relates to any one of the aforementioned ampoules, wherein the average outer diameter of the ampoule is between about 2,54mm (0.1") and about 101,6mm (0.4"). In certain arrangements, the present disclosure relates to any one of the aforementioned ampoules, wherein the average outer diameter of the ampoule is between about 10,6mm (0.4") and about 15,2mm (0.6"). In certain arrangements, the present disclosure relates to any one of the aforementioned ampoules, wherein the average outer diameter of the ampoule is between about 15,2mm (0.6") and about 20,3mm (0.8"). In certain arrangements, the present disclosure relates to any one of the aforementioned ampoules, wherein the average outer diameter of the ampoule is between about 20,3mm (0.8") and about 25,4mm (1"). In certain arrangements, the present disclosure relates to any one of the aforementioned ampoules, wherein the average outer diameter of the ampoule is about 6,604mm (0.26"). In certain arrangements, the present disclosure relates to any one of the aforementioned ampoules, wherein the average outer diameter of the ampoule is about 9,906mm (0.39"). In certain arrangements, the present disclosure to any one of the aforementioned ampoules, wherein the average outer diameter of the ampoule is about 13,97mm (0.55"). In certain arrangements, the present disclosure relates to anyone of the aforementioned ampoules, wherein the average outer diameter of the ampoule is about 24,638mm (0.97").

In certain arrangements, the present disclosure relates to any one of the aforementioned ampoules, wherein the ampoule is plastic.

In certain arrangements, the present disclosure relates to any one of the aforementioned ampoules, wherein the hydrophobic septum comprises a hydrophobic porous filter. In certain arrangements, the present disclosure relates to any one of the aforementioned ampoules, wherein the hydrophobic septum is a hydrophobic porous filter. In certain arrangements, the present disclosure relates to any one of the aforementioned ampoules, wherein the hydrophobic porous filter comprises a flat sheet, hollow fiber, or spiral wound membrane. In certain arrangements, the present disclosure relates to any one of the aforementioned ampoules, wherein the hydrophobic septum comprises polyethylene, polypropylene, polytetrafluoroethylene, polyvinylidene fluoride, polysulfone, polyethersulfone, polyvinyl chloride, or combinations thereof.

In certain arrangements, the present disclosure relates to any one of the aforementioned ampoules, wherein the hydrophobic septum is at least about 6,35mm (0,25") from the proximal end of the ampoule. In certain arrangements, the present disclosure relates to any one of the aforementioned ampoules, wherein the hydrophobic septum is at least about 15,7mm (0,5") from the proximal end of the ampoule. In certain arrangements, the present disclosure relates to any one of the aforementioned ampoules, wherein the hydrophobic septum is at least about 19,05mm (0,75") from the proximal end of the ampoule.

In certain arrangements, the present disclosure relates to any one of the aforementioned ampoules, wherein the inside of the bottom compartment is coated with the polymerizable component.

In certain arrangements, the present disclosure relates to any one of the aforementioned ampoules, further comprising a dissolution aid scaffold; wherein the polymerizable component is adsorbed onto the dissolution aid scaffold. In certain arrangement, the present disclosure relates to any one of the aforementioned ampoules, wherein the dissolution aid scaffold is a piece of porous material. In certain embodiment, the present invention relates to any one of the aforementioned ampoules, wherein the dissolution aid scaffold is a piece of porous material which is configured and adapted so that fluid passing through the bottom compartment and out the distal end of the ampoule passes through the piece of porous material and reconstitutes the polymerizable component adsorbed thereon.

In certain arrangements, the present disclosure relates to any one of the aforementioned ampoules, wherein the dissolution aid scaffold is a piece of plastic or glass.

In certain arrangements, the present disclosure relates to any one of the aforementioned ampoules, wherein the dissolution aid scaffold is a piece of polyethylene (PE), polypropylene (PP), high-density polyethylene (HDPE), ultra-high molecular weight polyethylene (UHMWPE), polytetrafluoroethylene (PTFE), nylon, polyvinylidine fluoride (PVDF), polyethersulfone (PES), ethyl vinyl acetate (EVA), or a co-polymer thereof. In certain arrangements, the present disclosure relates to any one of the aforementioned ampoules, wherein the dissolution aid scaffold is a piece of polyester batting or other fibrous filter media material.

In certain arrangements, the present disclosure relates to any one of the aforementioned ampoules, wherein the dissolution aid scaffold is shaped like a disc and has a diameter equal to that of the ampoule.

In certain arrangements, the present disclosure relates to any one of the aforementioned ampoules, wherein the dissolution aid scaffold is at least about 6,35mm (0,25") from the distal end of the ampoule. In certain arrangements, the present disclosure relates to any one of the aforementioned ampoules, wherein the dissolution aid scaffold is at least about 12,7mm (0,5") from the distal end of the ampoule. In certain arrangements, the present disclosure relates to any one of the aforementioned ampoules, wherein the dissolution aid scaffold is at least about 19,05mm (0,75") from the distal end of the ampoule.

In certain arrangements, the present disclosure relates to any one of the aforementioned ampoules, wherein instead of a hydrophobic septum, a floating plunger head is used, wherein the floating plunger head is positioned between the proximal and distal ends of the ampoule, thereby creating a top compartment, bounded by the proximal end of the syringe and the floating plunger head, and a bottom compartment, bounded by the distal end of the syringe and the floating plunger head; wherein the floating plunger head contains a check valve which, when opened, connects the top compartment and the bottom compartment; the floating plunger head is configured and adapted to form a seal with the sidewall of the ampoule; and the floating plunger is axially translatable from a position near the proximal end of the ampoule to a position at the distal end of the ampoule.

In certain arrangements, the present disclosure relates to any one of the aforementioned ampoules, wherein instead of a hydrophobic septum, a floating plunger head is used, wherein the floating plunger head is positioned between the proximal and distal ends of the ampoule, thereby creating a top compartment, bounded by the proximal end of the syringe and the floating plunger head, and a bottom compartment, bounded by the distal end of the syringe and the floating plunger head; wherein the floating plunger head contains a frangible membrane which, when broken, connects the top compartment and the bottom compartment; the floating plunger head is configured and adapted to form a seal with the sidewall of the ampoule; and the floating plunger is axially translatable from a position near the proximal end of the ampoule to a position at the distal end of the ampoule.

Another aspect of the present disclosure, not part of the present invention relates to an applicator, comprising:
a syringe body having a sidewall, and an opening at each of its proximal and distal ends;
a floating plunger head positioned between the proximal and distal ends of the syringe body, thereby creating a top compartment, bounded by the proximal end of the syringe and the floating plunger head, and a bottom compartment, bounded by the distal end of the syringe and the floating plunger head; wherein the floating plunger head contains a check valve which, when opened, connects the top compartment and the bottom compartment; the floating plunger head is configured and adapted to form a seal with the sidewall of the syringe body; and the floating plunger is axially translatable from a position near the proximal end of the syringe body to a position at the distal end of the syringe body;
a plunger having a shaft, a distal end, a proximal end, and a plunger head provided at the distal end; the plunger inserted into the syringe body plunger head first through the proximal opening of the syringe body; the plunger head configured and adapted to form a seal when the plunger head engages the sidewall of the syringe body, wherein the plunger is axially translatable between a position where the plunger head is at the proximal end of the syringe body and a position wherein the plunger head is flush with the floating plunger at the distal end of the syringe body;
a bladder having an opening at each of its proximal and distal ends;
an adaptor which creates a fluid path from the distal end of the syringe body to the proximal end of the bladder;
a tip having an opening at each of its proximal and distal ends, wherein the distal end of the bladder is connected to the proximal end of the tip;
a liquid in the top compartment;
a polymerizable component in the bottom compartment or in the adaptor; and
a solid in the bladder.

In certain arrangements, the present disclosure relates to any one of the aforementioned applicators, wherein the liquid in the top compartment is water. In certain embodiments, the present invention relates to any one of the aforementioned applicators, wherein the liquid in the top compartment is buffer. In certain arrangements, the present disclosure relates to any one of the aforementioned applicators, wherein the liquid in the top compartment is an aqueous solution which comprises phosphates. In certain arrangements, the present disclosure relates to any one of the aforementioned applicators, wherein the liquid in the top compartment is an aqueous solution which comprises borates.

In certain arrangements, the present disclosure relates to any one of the aforementioned applicators, wherein the syringe body further comprises a liquid in the bottom compartment. In certain arrangements, the present disclosure relates to any one of the aforementioned applicators, wherein the liquid in the bottom compartment is a concentrated salt solution, which when diluted forms a buffer. In certain arrangements, the present disclosure relates to any one of the aforementioned applicators, wherein the liquid in the bottom compartment is an aqueous solution which comprises phosphates. In certain arrangements, the present disclosure relates to any one of the aforementioned applicators, wherein the liquid in the bottom compartment is an aqueous solution which comprises borates.

In certain arrangements, the present disclosure relates to any one of the aforementioned applicators, wherein the polymerizable material comprises a polyalkyleneimine. In certain arrangements, the present disclosure relates to any one of the aforementioned applicators, wherein the polymerizable material comprises PEI.

In certain arrangements, the present disclosure relates to any one of the aforementioned applicators, wherein the solid inside the bladder comprises an activated PEG. In certain arrangements, the present disclosure relates to any one of the aforementioned applicators, wherein the solid inside the bladder comprises a PEG-(NHS)₂ reagent. In certain arrangements, the present disclosure relates to any one of the aforementioned applicators, wherein the solid inside the bladder comprises PEG-SPA, PEG-SSeb, PEG-SG or a mixture thereof. In certain arrangements, the present disclosure relates to any one of the aforementioned applicators, wherein the solid inside the bladder comprises PEG-SSeb. In certain arrangements, the present disclosure relates to any one of the aforementioned applicators, wherein the solid inside the bladder comprises PEG-SPA.

In certain arrangements, the present disclosure relates to any one of the aforementioned applicators, wherein the syringe body is tubular. In certain embodiments, the present invention relates to any one of the aforementioned applicators, wherein the syringe body is plastic.

In certain arrangements, the present disclosure relates to any one of the aforementioned applicators, wherein the length of the syringe body is between about 25,4mm (1") and about 127mm (5"). In certain arrangements, the present disclosure relates to any one of the aforementioned applicators, wherein the length of the syringe body is between about 50,8mm (2") and about 101,6mm (4"). In certain arrangements, the present disclosure relates to any one of the aforementioned applicators, wherein the length of the syringe body is about 76,2mm (3").

In certain arrangements, the present disclosure relates to any one of the aforementioned applicators, wherein the length of the syringe body is about 50,88mm (2"). In certain arrangements, the present disclosure relates to any one of the aforementioned applicators, wherein the length of the syringe body is about 25,4mm (1"). In certain arrangements, the present disclosure relates to any one of the aforementioned applicators, wherein the average outer diameter of the syringe body is between about 2,54mm (0.1") and about 25,4mm (1"). In certain arrangements, the present disclosure relates to any one of the aforementioned applicators, wherein the average outer diameter of the syringe body is between about 2,54mm (0.1") and about 101,6mm (0.4"). In certain arrangements, the present disclosure relates to any one of the aforementioned applicators, wherein the average outer diameter of the syringe body is between about 10,6mm (0.4") and about 15,2mm (0.6"). In certain arrangements, the present disclosure relates to any one of the aforementioned applicators, wherein the average outer diameter of the syringe body is between about 15,2mm (0.6") and about 20,3mm (0.8"). In certain arrangements, the present disclosure relates to any one of the aforementioned applicators, wherein the average outer diameter of the syringe body is between about 20,3mm (0.8") and about 25,4mm (1"). In certain arrangements, the present disclosure relates to any one of the aforementioned applicators, wherein the average outer diameter of the syringe body is about 6,604mm (0.26"). In certain arrangements, the present disclosure relates to any one of the aforementioned applicators, wherein the average outer diameter of the syringe body is about 9,906mm (0.39"). In certain arrangements, the present disclosure relates to any one of the aforementioned applicators, wherein the average outer diameter of the syringe body is about 13,97mm (0.55"). In certain arrangements, the present disclosure relates to any one of the aforementioned applicators, wherein the average outer diameter of the syringe body is about 24,638mm (0.97").

In certain embodiments, the present invention relates to any one of the aforementioned syringes, further comprising a dissolution aid scaffold; wherein the polymerizable component is adsorbed onto the dissolution aid scaffold. In certain embodiments, the present invention relates to any one of the aforementioned syringes, wherein the dissolution aid scaffold is a piece of porous material. In certain embodiments, the present invention relates to any one of the aforementioned syringes, wherein the dissolution aid scaffold is a piece of porous material which is configured and adapted so that fluid passing through the bottom compartment and out the distal end of the syringe body passes through the piece of porous material and reconstitutes the polymerizable component adsorbed thereon.

In certain embodiments, the present invention relates to any one of the aforementioned syringes, wherein the dissolution aid scaffold is a piece of plastic or glass. In certain embodiments, the present invention relates to any one of the aforementioned syringes, wherein the dissolution aid scaffold is a piece of polyethylene (PE), polypropylene (PP), high-density polyethylene (HDPE), ultra-high molecular weight polyethylene (UHMWPE), polytetrafluoroethylene (PTFE), nylon, polyvinylidine fluoride (PVDF), polyethersulfone (PES), ethyl vinyl acetate (EVA), or a co-polymer thereof. In certain embodiments, the present invention relates to any one of the aforementioned syringes, wherein the dissolution aid scaffold is a piece of polyester batting or other fibrous filter media material.

In certain embodiments, the present invention relates to any one of the aforementioned syringes, wherein the dissolution aid scaffold is shaped like a disc and has a diameter equal to that of the syringe body.

In certain embodiments, the present invention relates to any one of the aforementioned syringes, wherein the dissolution aid scaffold is shaped like a disc and has a diameter equal to that of the adaptor.

In certain arrangements, the present disclosure relates to any one of the aforementioned applicators, wherein the floating plunger is at least about 6.35 mm (0.25") from the proximal end of the syringe body. In certain arrangements, the present disclosure relates to any one of the aforementioned applicators, wherein the floating plunger is at least about 12.7mm (0,5") from the proximal end of the syringe body. In certain arrangements, the present disclosure relates to any one of the aforementioned applicators, wherein the floating plunger is at least about 19,05mm (0,75") from the proximal end of the syringe body.

In certain arrangements, the present disclosure relates to any one of the aforementioned applicators, wherein the check valve is pressure sensitive. In certain arrangements, the present disclosure relates to any one of the aforementioned applicators, wherein the check valve is a ball check valve, a diaphragm check valve, a swing check valve, a lift-check valve, a double check valve, a double ball check valves, a piston check valve, a wafer check valve, a duckbill check valve, an umbrella check valve, or a ball-and-cone check valve.

In certain arrangements, the present disclosure relates to any one of the aforementioned applicators, wherein instead of a check valve, the applicator has a bypass built into the outer part of the syringe body, thereby accomplishing the same function. In certain arrangements, the present disclosure relates to any one of the aforementioned applicators, wherein instead of a check valve, the floating plunger has frangible membrane, which when broken allows for fluid communication between the top and bottom compartments, thereby accomplishing the same function.

In certain arrangements, the present disclosure relates to any one of the aforementioned applicators, wherein the bladder is tubular.

In certain arrangements, the present disclosure relates to any one of the aforementioned applicators, wherein the bladder is made from a compressible material, wherein the compressible material permits manual pressurization. In certain arrangements, the present disclosure relates to any one of the aforementioned applicators, wherein the bladder is plastic.

In certain arrangements, the present disclosure relates to any one of the aforementioned applicators, wherein the length of the bladder is between about 25,4mm (1") and about 127mm (5").

In certain arrangements, the present disclosure relates to any one of the aforementioned applicators, wherein the length of the bladder is between 50,8mm (2") and about 101,6mm (4").

In certain arrangements, the present disclosure relates to any one of the aforementioned applicators, wherein the length of the bladder is about 76,2mm (3").

In certain arrangements, the present disclosure relates to any one of the aforementioned applicators, wherein the length of the bladder is about 50,88mm (2"). In certain arrangements, the present disclosure relates to any one of the aforementioned applicators, wherein the length of the bladder is about 25,4mm (1").

In certain arrangements, the present disclosure relates to any one of the aforementioned applicators, wherein the average outer diameter of the bladder is between about 2,54mm (0.1") and about 25,4mm (1"). In certain arrangements, the present disclosure relates to any one of the aforementioned applicators, wherein the average outer diameter of the bladder is between about 2,54mm (0.1") and about 7,62mm (0.3"). In certain arrangements, the present disclosure relates to any one of the aforementioned applicators, wherein the average outer diameter of the bladder is between about 7,62mm (0.3") and about 12,7mm (0.5"). In certain arrangements, the present disclosure relates to any one of the aforementioned applicators, wherein the average outer diameter of the bladder is between about 12,7mm (0.5") and about 17,78mm (0.7"). In certain arrangements, the present disclosure relates to any one of the aforementioned applicators, wherein the average outer diameter of the bladder is between about 17,78mm (0.7") and about 25,4mm (1").

In certain arrangements, the present disclosure relates to any one of the aforementioned applicators, wherein the adaptor is tubular. In certain arrangements, the present disclosure relates to any one of the aforementioned applicators, wherein the adaptor is plastic.

In certain arrangements, the present disclosure relates to any one of the aforementioned applicators, wherein the average wall thickness of the bladder is between about 0,0254mm (0.001") and about 12,7mm (0.5"). In certain arrangements, the present disclosure relates to any one of the aforementioned applicators, wherein the average wall thickness of the bladder is between about 0,0254mm (0.001") and about 0,254mm (0.01"). In certain arrangements, the present disclosure relates to any one of the aforementioned applicators, wherein the average wall thickness of the bladder is between about 0,254mm (0.01") and about 2,54mm (0.1"). In certain arrangements, the present disclosure relates to any one of the aforementioned applicators, wherein the average wall thickness of the bladder is between about 2,54mm (0.1") and about 12,7mm (0.5"). In certain arrangements, the present disclosure relates to any one of the aforementioned applicators, wherein the length of the adaptor is between about 2,54mm (0.1") and about 50,8mm (2").

In certain arrangements, the present disclosure relates to any one of the aforementioned applicators, wherein the length of the adaptor is between about 2.54mm (0.1") and about 12,7mm (0.5"). In certain arrangements, the present disclosure relates to any one of the aforementioned applicators, wherein the length of the adaptor is between about 12,7mm (0.5") and 25,4mm (1"). In certain arrangements, the present disclosure relates to any one of the aforementioned applicators, wherein the length of the adaptor is between about 25,4mm (1") and about 38,1mm (1.5"). In certain arrangements, the present disclosure relates to any one of the aforementioned applicators, wherein the length of the adaptor is between about 38,1mm (1.5") and 50,8mm (2").

In certain arrangements, the present disclosure relates to any one of the aforementioned applicators, further comprising a removable separator or valve at the distal end of the syringe body that controls fluid flow from syringe body to the adaptor.

In certain arrangements, the present disclosure relates to any one of the aforementioned applicators, wherein the tip is a cannula tip, a bent cannula tip, a bayonet¬ style cannula tip, a brush applicator tip, a duck-bill tip, a textile mitt, a spray tip, a foam swab, or a silicon tube. In certain arrangements, the present disclosure relates to any one of the aforementioned applicators, wherein the tip is a bent cannula tip or a bayonet-style cannula tip.

In certain arrangements, the present disclosure relates to any one of the aforementioned applicators, wherein the length of the tip is between about 2,54mm (0.1") and about 254mm (10"). In certain arrangements, the present disclosure relates to any one of the aforementioned applicators, wherein the length of the tip is between about 25,4mm (1") and about 50,8mm (2"). In certain arrangements, the present disclosure relates to any one of the aforementioned applicators, wherein the length of the tip is between about 50,8mm (2") and about 76,2mm (3"). In certain embodiments, the present invention relates to any one of the aforementioned applicators, wherein the length of the tip is between about 76,2mm (3") and about 101,6mm (4"). In certain arrangements, the present disclosure relates to any one of the aforementioned applicators, wherein the length of the tip is between about 101,6,, (4") and about 127mm (5"). In certain arrangements, the present disclosure relates to any one of the aforementioned applicators, wherein the length of the tip is between about 127mm (5") and about 152,4mm (6"). In certain arrangements, the present disclosure relates to any one of the aforementioned applicators, wherein the length of the tip is between about 152,4mm (6") and about 177,8mm (7"). In certain arrangements, the present disclosure relates to any one of the aforementioned applicators, wherein the length of the tip is between about 177,8mm (7") and about 203,2mm (8"). In certain arrangements, the present disclosure relates to any one of the aforementioned applicators, wherein the length of the tip is between about 203,2mm (8") and about 228,6mm (9").

In certain embodiments, the present invention relates to any one of the aforementioned applicators, wherein the length of the tip is between about 228,6mm (9") and about 254mm(10").

In certain arrangements, the present disclosure relates to any one of the aforementioned applicators, wherein the diameter of the distal end of the tip is between about 0.381mm (0.015") and about 1.397mm (0.55"). In certain arrangements, the present disclosure relates to any one of the aforementioned applicators, wherein the diameter of the distal end of the tip is between about 0.381mm (0.015") and about 5.08mm (0.2"). In certain arrangements, the present disclosure relates to any one of the aforementioned applicators, wherein the diameter of the distal end of the tip is between about 0.889mm (0.035") and 3.81mm (0.15"). In certain arrangements, the present disclosure relates to any one of the aforementioned applicators, wherein the diameter of the distal end of the tip is between about 0.1397mm (0.055") and 2.54mm (0.1"). In certain arrangements, the present disclosure relates to any one of the aforementioned applicators, wherein the diameter of the distal end of the tip is about 2.032mm (0.08").

In certain embodiments, the present invention relates to any one of the aforementioned applicators, further comprising a screen or filter placed in the tip.

As is apparent from the description of the plural embodiments above, remarkable multi-component syringes and applicator assemblies are herein provided. While such multi-component syringes and applicators can be used for dispensing hydrogels, the present invention is also applicable to the dispensing any plural component mixtures which are to be mixed prior to use and which may need to be stored separately. In particular, the present invention includes syringes for concurrently dispensing plural flowable component materials, which overcomes shortcomings associated with known multiple component dispensers. For example, the present invention is applicable to dispensing two-component adhesives, sealants, coatings, and potting compounds, such as epoxies, urethanes, acrylics, polysulfides, polyesters, and silicones, as well as other adhesive or sealant materials and the like.

Epoxy adhesives are an example of a multiple-component adhesive, and more generally of a multiple-component product, that requires the component materials to be stored individually prior to use, and which must be mixed in accordance with a specific ratio prior to use. Epoxy adhesives typically include an epoxy-resin component and a curing-agent component, such that when mixed together in proper proportions, the epoxy curative hardens in place. With this in mind, the applicators described herein may be used as epoxy-adhesive dispensing-devices, having separate storage locations for the epoxy-resin component and the curing-agent component, and a means for dispensing together the separate components and in accordance with the proper ratio for curing.

In addition, a variety of polyurethanes, such as those which are useful for bonding to porous and non-porous substrates, also requires the component materials to be stored individually and mixed in accordance with a specific ratio for usage. Polyurethane adhesive compositions typically comprise at least one polyurethane prepolymer and a curing agent.

Further, fibrin sealants, which are formed from blood plasma components, comprise a first component containing fibrinogen and Factor XIII, and a second component, which usually includes thrombin and calcium ions, may also be delivered via the applicators disclosed herein. It is well known that fibrinogen is capable of polymerizing and being cross-linked to form a fibrin clot when the proper components are mixed. The necessary additional factors to simulate relevant portions of the natural blood coagulation cascade can be suitably distributed between the fibrinogen and thrombin components. See, for example, Antanavich et al. United States Patent No. 5,585,007, provides an extensive discussion of fibrinogen sealant preparation (column 1, line 20 to column 4, line 62) and applicators (column 4 line 62 to column 5, line 14).

In addition, in certain embodiments, to facilitate the mixing of the components in the applicator, the applicator parts can be independently caped. In certain embodiments the caps may or may not allow for the passage of air through the cap. An airtight cap, wherein no material can be injected into the cap, is referred to as "airlocked." Caps which allow the passage of air, as described above, are there to cover the given end in a "non-airtight" manner. Caps can also be designed so as to allow air to only flow in one direction.

### SELECTED METHODS

One aspect of the disclosure, not part of the present invention, relates to a method of preparing and applying a hydrogel to a surface; comprising the steps of:
(a) obtaining a syringe comprising:
   a syringe body having a sidewall, and an opening at each of its proximal and distal ends;
   a floating hydrophobic septum contacting the sidewall of the syringe body and translatable from a position near the proximal end of the syringe body to a position at the distal end of the syringe body, thereby creating a top compartment, bounded by the proximal end of the syringe and the hydrophobic septum, and a bottom compartment, bounded by the distal end of the syringe and the hydrophobic septum;
   a liquid in the top compartment;
   a polymerizable component in the bottom compartment; and
   a plunger having a shaft, a distal end, a proximal end, and a plunger head provided at the distal end; the plunger inserted into the syringe body plunger head first through the proximal opening of the syringe body; the plunger head configured and adapted to form a seal when the plunger head engages the sidewall of the syringe body, wherein the plunger is axially translatable between a position where the plunger head is at the proximal end of the syringe body and a position wherein the plunger head is flush with the hydrophobic septum;
(b) advancing the plunger head towards the hydrophobic septum, causing the liquid in the top compartment to pass through the hydrophobic septum and into the bottom compartment, thereby dissolving the polymerizable component;
(c) retracting the plunger back towards the proximal end of the syringe body;
(d) assembling an apparatus comprising the syringe with a dissolved polymerizable component, an adaptor, a bladder and a tip; wherein the bladder has an opening at each of its proximal and distal ends; there is a solid in the bladder; the tip has an opening at each of its proximal and distal ends; the adaptor connects the distal end of the syringe body to the proximal end of the bladder; and the distal end of the bladder is connected to the proximal end of the tip;
(e) advancing the plunger head towards the proximal end of the syringe body, causing the hydrophobic septum to move to the distal end of the syringe body and causing the liquid comprising the dissolved polymerizable component to pass through the adaptor and into contact with the solid in the bladder, thereby forming a pre-hydrogel mixture; and
(f) applying the liquid pre-hydrogel mixture to the surface via the tip, wherein the pre-hydrogel mixture gels to form a hydrogel on the surface.

In certain arrangements, the present disclosure relates to any one of the aforementioned methods, wherein the liquid pre-hydrogel mixture is applied by manually compressing the bladder to force the pre-hydrogel mixture out of the bladder, into the tip, and onto the surface.

In certain arrangements, the present disclosure relates to any one of the aforementioned methods, wherein the liquid pre-hydrogel mixture is applied by retracting the plunger back toward the proximal end of the syringe body and subsequently advancing the plunger to force the pre-hydrogel mixture out of the bladder, into the tip, and onto the surface.

In certain arrangements, the present disclosure relates to any one of the aforementioned methods, wherein the surface is dura mater. In certain arrangements, the present disclosure relates to any one of the aforementioned methods, wherein the surface is dura mater within the spinal column. In certain arrangements, the present disclosure relates to any one of the aforementioned methods, wherein the surface is cardiovascular tissue. In certain arrangements, the present disclosure relates to any one of the aforementioned methods, wherein the surface is gall tissue. In certain arrangements, the present disclosure relates to any one of the aforementioned methods, wherein the surface is urinary bladder tissue.

In certain arrangements, the present disclosure relates to any one of the aforementioned methods, further comprising a cap, luer lock or frangible seal at the distal end of the syringe body. In certain arrangements, the present disclosure relates to any one of the aforementioned methods, further comprising a cap at the distal end of the syringe body. In certain arrangements, the present disclosure relates to any one of the aforementioned methods, further comprising removing the cap before assembling the apparatus. In certain arrangements, the present disclosure relates to any one of the aforementioned methods, further comprising the step of opening the luer lock after the assembly of the apparatus. In certain arrangements, the present disclosure relates to any one of the aforementioned methods, wherein the adaptor ruptures the frangible seal upon assembly.

In certain arrangements, the present disclosure relates to any one of the aforementioned methods, wherein the liquid in the top compartment is a buffer.

In certain arrangements, the present disclosure relates to any one of the aforementioned methods, wherein the polymerizable component comprises a polyalkyleneimine. In certain arrangements, the present disclosure relates to any one of the aforementioned methods, wherein the polymerizable component comprises PEI.

In certain arrangements, the present disclosure relates to any one of the aforementioned methods, wherein the bottom compartment further comprises a liquid so that the polymerizable component is about 20% w/w; about 30% w/w; about 40% w/w; about 50% w/w; about 60% w/w; about 70% w/w; about 90% w/w; or about 90% w/w.

In certain arrangements, the present disclosure relates to any one of the aforementioned methods, wherein the syringe body is tubular. In certain arrangements, the present disclosure relates to any one of the aforementioned methods, wherein the syringe body is plastic.

In certain arrangements, the present disclosure relates to any one of the aforementioned methods, wherein the length of the syringe body is between about 25,4mm (1") and about 127mm (5"). In certain arrangements, the present disclosure relates to any one of the aforementioned methods, wherein the length of the syringe body is between about 50,8mm (2") and about 101,6mm (4"). In certain arrangements, the present disclosure relates to any one of the aforementioned methods, wherein the length of the syringe body is about 76,2mm (3"). In certain arrangements, the present disclosure relates to any one of the aforementioned methods, wherein the length of the syringe body is about 50,8mm (2"). In certain embodiments, the present invention relates to any one of the aforementioned methods, wherein the length of the syringe body is about 25.4mm (1").

In certain arrangements, the present disclosure relates to any one of the aforementioned methods, wherein the average outer diameter of the syringe body is between about 2,54mm (0.1") and about 25,4mm (1"). In certain arrangements, the present disclosure relates to any one of the aforementioned methods, wherein the average outer diameter of the syringe body is between about 2,54mm (0.1") and about 10,16mm (0.4"). In certain arrangements, the present disclosure relates to any one of the aforementioned methods, wherein the average outer diameter of the syringe body is between about 10,16mm (0.4") and about 15,24mm (0.6"). In certain arrangements, the present disclosure relates to any one of the aforementioned methods, wherein the average outer diameter of the syringe body is between about 15,24mm (0.6") and about 20,32mm (0.8"). In certain arrangements, the present disclosure relates to any one of the aforementioned methods, wherein the average outer diameter of the syringe body is between about 20.32mm (0.8") and about 25,4mm (1").In certain arrangements, the present disclosure relates to any one of the aforementioned methods, wherein the average outer diameter of the syringe body is about 6,604mm (0.26"). In certain arrangements, the present disclosure relates to any one of the aforementioned methods, wherein the average outer diameter of the syringe body is about 9,906mm (0.39"). In certain arrangements, the present disclosure relates to any one of the aforementioned methods, wherein the average outer diameter of the syringe body is about 13,97mm (0.55"). In certain embodiments, the present invention relates to any one of the aforementioned methods, wherein the average outer diameter of the syringe body is about 24,638mm (0,97").

In certain arrangements, the present disclosure relates to any one of the aforementioned methods, wherein the hydrophobic septum comprises a hydrophobic porous filter. In certain arrangements, the present disclosure relates to any one of the aforementioned methods, wherein the hydrophobic septum is a hydrophobic porous filter. In certain embodiments, the present invention relates to any one of the aforementioned methods, wherein the hydrophobic porous filter comprises a flat sheet, hollow fiber, or spiral wound membrane. In certain arrangements, the present disclosure relates to any one of the aforementioned methods, wherein the hydrophobic septum comprises polyethylene, polypropylene, polytetrafluoroethylene, polyvinylidene fluoride, polysulfone, polyethersulfone, polyvinyl chloride, or combinations thereof.

In certain arrangements, the present disclosure relates to any one of the aforementioned methods, wherein the hydrophobic septum is at least about 6,35mm (0,25") ' from the proximal end of the syringe body. In certain arrangements, the present disclosure relates to any one of the aforementioned methods, wherein the hydrophobic septum is at least about 12,7mm (0,5") from the proximal end of the syringe body. In certain arrangements, the present disclosure relates to any one of the aforementioned methods, wherein the hydrophobic septum is at least about 19,05mm (0,75") ' from the proximal end of the syringe body.

In certain arrangements, the present disclosure relates to any one of the aforementioned methods, wherein the inside of the bottom compartment is coated with the polymerizable component.

In certain arrangements, the present disclosure relates to any one of the aforementioned methods, further comprising a dissolution aid scaffold; wherein the polymerizable component is adsorbed onto the dissolution aid scaffold. In certain arrangements, the present disclosure relates to any one of the aforementioned methods, wherein the dissolution aid scaffold is a piece of porous material. In certain arrangements, the present disclosure relates to any one of the aforementioned methods, wherein the dissolution aid scaffold is a piece of porous material which is configured and adapted so that fluid passing through the bottom compartment and out the distal end of the syringe body passes through the piece of porous material and reconstitutes the polymerizable component adsorbed thereon.

In certain arrangements, the present disclosure relates to any one of the aforementioned methods, wherein the dissolution aid scaffold is a piece of plastic or glass. In certain embodiments, the present invention relates to any one of the aforementioned methods, wherein the dissolution aid scaffold is a piece of polyethylene (PE), polypropylene (PP), high-density polyethylene (HDPE), ultra-high molecular weight polyethylene (UHMWPE), polytetrafluoroethylene (PTFE), nylon, polyvinylidine fluoride (PVDF), polyethersulfone (PES), ethyl vinyl acetate (EVA), or a co-polymer thereof. In certain arrangements, the present disclosure relates to any one of the aforementioned methods, wherein the dissolution aid scaffold is a piece of polyester batting or other fibrous filter media material.

In certain arrangements, the present disclosure relates to any one of the aforementioned methods, wherein the dissolution aid scaffold is shaped like a disc and has a diameter equal to that of the syringe body.

In certain arrangements, the present disclosure relates to any one of the aforementioned methods, wherein the dissolution aid scaffold is at least about 6,35mm (0,25") from the distal end of the syringe body. In certain arrangements, the present disclosure relates to any one of the aforementioned methods, wherein the dissolution aid scaffold is at least about 12,7mm (0,5") from the distal end of the syringe body. In certain arrangements, the present disclosure relates to any one of the aforementioned methods, wherein the dissolution aid scaffold is at least about 19,05mm (0,75") from the distal end of the syringe body.

In certain arrangements, the present disclosure relates to any one of the aforementioned methods, wherein instead of a hydrophobic septum, a floating plunger head is used, wherein the floating plunger head is positioned between the proximal and distal ends of the syringe body, thereby creating a top compartment, bounded by the proximal end of the syringe and the floating plunger head, and a bottom compartment, bounded by the distal end of the syringe and the floating plunger head; wherein the floating plunger head contains a check valve which, when opened, connects the top compartment and the bottom compartment; the floating plunger head is configured and adapted to form a seal with the sidewall of the syringe body; and the floating plunger is axially translatable from a position near the proximal end of the syringe body to a position at the distal end of the syringe body.

In certain arrangements, the present disclosure relates to any one of the aforementioned methods, wherein instead of a hydrophobic septum, a floating plunger head is used, wherein the floating plunger head is positioned between the proximal and distal ends of the syringe body, thereby creating a top compartment, bounded by the proximal end of the syringe and the floating plunger head, and a bottom compartment, bounded by the distal end of the syringe and the floating plunger head; wherein the floating plunger head contains a frangible membrane which, when broken, connects the top compartment and the bottom compartment; the floating plunger head is configured and adapted to form a seal with the sidewall of the syringe body; and the floating plunger is axially translatable from a position near the proximal end of the syringe body to a position at the distal end of the syringe body.

One aspect of the disclosure not part of the present invention relates to a method of preparing and applying a hydrogel to a surface; comprising the steps of:
(a) obtaining an ampoule comprising:
   a closed proximal end and a frangible seal at its distal end;
   a floating hydrophobic septum positioned inside the ampoule so as to create a top compartment, bounded by the sealed proximal end of the ampoule and the hydrophobic septum, and a bottom compartment, bounded by the frangible seal at the distal end of the ampoule and the hydrophobic septum;
   a liquid in the top compartment; and
   a polymerizable component in the bottom compartment;
(b) squeezing the ampoule outside of the top compartment, causing the liquid in the top compartment to pass through the hydrophobic septum and into the bottom compartment, thereby dissolving the polymerizable component;
(c) assembling an apparatus comprising the ampoule with a dissolved polymerizable component, an adaptor, a bladder and a tip; wherein the bladder has an opening at each of its proximal and distal ends; there is a solid in the bladder; the tip has an opening at each of its proximal and distal ends; the adaptor breaks the frangible seal on the distal end of the ampoule and thereby connects the distal end of the ampoule to the proximal end of the bladder; and the distal end of the bladder is connected to the proximal end of the tip;
(d) squeezing the ampoule outside the bottom compartment, causing the liquid comprising the dissolved polymerizable component to pass through the adaptor and into contact with the solid in the bladder, thereby forming a pre-hydrogel mixture; and
(e) applying the liquid pre-hydrogel mixture to the surface via the tip, wherein the pre-hydrogel mixture gels to form a hydrogel on the surface.

In certain arrangements, the present disclosure relates to any one of the aforementioned methods, wherein the liquid pre-hydrogel mixture is applied by manually compressing the bladder to force the pre-hydrogel mixture out of the bladder, into the tip, and onto the surface.

In certain arrangements, the present disclosure relates to any one of the aforementioned methods, wherein the surface is dura mater. In certain arrangements, the present disclosure relates to any one of the aforementioned methods, wherein the surface is dura mater within the spinal column. In certain arrangements, the present disclosure relates to any one of the aforementioned methods, wherein the surface is cardiovascular tissue. In certain arrangements, the present disclosure relates to any one of the aforementioned methods, wherein the surface is gall tissue. In certain arrangements, the present disclosure relates to any one of the aforementioned methods, wherein the surface is urinary bladder tissue.

In certain arrangements, the present disclosure relates to any one of the aforementioned methods, wherein the liquid in the top compartment is a buffer.

In certain arrangements, the present disclosure relates to any one of the aforementioned methods, wherein the polymerizable component comprises a polyalkyleneimine. In certain arrangements, the present disclosure relates to any one of the aforementioned methods, wherein the polymerizable component comprises PEI.

In certain arrangements, the present disclosure relates to any one of the aforementioned methods, wherein the bottom compartment further comprises a liquid so that the polymerizable component is about 20% w/w; about 30% w/w; about 40% w/w; about 50% w/w; about 60% w/w; about 70% w/w; about 90% w/w; or about 90% w/w.

In certain arrangements, the present disclosure relates to any one of the aforementioned methods, wherein the ampoule is tubular. In certain arrangements, the present disclosure relates to any one of the aforementioned methods, wherein the ampoule is plastic.

In certain arrangements, the present disclosure relates to any one of the aforementioned methods, wherein the length of the ampoule is between about 25,4mm (1") and about 127mm (5"). In certain arrangements, the present disclosure relates to any one of the aforementioned methods, wherein the length of the ampoule is between about 50,8mm (2") and about 101,6mm (4"). In certain arrangements, the present disclosure relates to any one of the aforementioned methods, wherein the length of the ampoule is about 76,2mm (3"). In certain arrangements, the present disclosure relates to any one of the aforementioned methods, wherein the length of the ampoule is about 50,8mm (2"). In certain arrangements, the present disclosure relates to any one of the aforementioned methods, wherein the length of the ampoule is about 25,4mm (1").

In certain arrangements, the present disclosure relates to any one of the aforementioned methods, wherein the average outer diameter of the ampoule is between about 2,54mm (0.1") and about 25,4mm (1"). In certain arrangements, the present disclosure relates to any one of the aforementioned methods, wherein the average outer diameter of the ampoule is between about 2,54mm (0.1") and about 101,6mm (0.4"). In certain arrangements, the present disclosure relates to any one of the aforementioned methods, wherein the average outer diameter of the ampoule is between about 10,6mm (0.4") and about 15,2mm (0.6"). In certain arrangements, the present disclosure relates to any one of the aforementioned methods, wherein the average outer diameter of the ampoule is between about 15,2mm (0.6") and about 20,3mm (0.8"). In certain arrangements, the present disclosure relates to any one of the aforementioned methods, wherein the average outer diameter of the ampoule is between about 20,3mm (0.8") and about 25,4mm (1"). In certain arrangements, the present disclosure relates to any one of the aforementioned methods, wherein the average outer diameter of the ampoule is about 6,604mm (0.26"). In certain arrangements, the present disclosure relates to any one of the aforementioned methods, wherein the average outer diameter of the ampoule is about 9,906mm (0.39"). In certain arrangements, the present disclosure to any one of the aforementioned methods, wherein the average outer diameter of the ampoule is about 13,97mm (0.55"). In certain arrangements, the present disclosure relates to anyone of the aforementioned methods, wherein the average outer diameter of the ampoule is about 24,638mm (0.97").

In certain arrangements, the present disclosure relates to any one of the aforementioned methods, wherein the ampoule is plastic.

In certain arrangements, the present disclosure relates to any one of the aforementioned methods, wherein the hydrophobic septum comprises a hydrophobic porous filter. In certain arrangements, the present disclosure relates to any one of the aforementioned methods, wherein the hydrophobic septum is a hydrophobic porous filter. In certain arrangements, the present disclosure relates to any one of the aforementioned methods, wherein the hydrophobic porous filter comprises a flat sheet, hollow fiber, or spiral wound membrane. In certain arrangements, the present disclosure relates to any one of the aforementioned methods, wherein the hydrophobic septum comprises polyethylene, polypropylene, polytetrafluoroethylene, polyvinylidene fluoride, polysulfone, polyethersulfone, polyvinyl chloride, or combinations thereof.

In certain arrangements, the present disclosure relates to any one of the aforementioned methods, wherein the hydrophobic septum is at least about 6,35mm (0,25") from the proximal end of the ampoule. In certain arrangements, the present disclosure relates to any one of the aforementioned methods, wherein the hydrophobic septum is at least about 12,7mm (0,50") from the proximal end of the ampoule. In certain arrangements, the present disclosure relates to any one of the aforementioned methods, wherein the hydrophobic septum is at least about 19,05mm (0,75") from the proximal end of the ampoule.

In certain arrangements, the present disclosure relates to any one of the aforementioned methods, wherein the inside of the bottom compartment is coated with the polymerizable component.

In certain arrangements, the present disclosure relates to any one of the aforementioned methods, further comprising a dissolution aid scaffold; wherein the polymerizable component is adsorbed onto the dissolution aid scaffold. In certain arrangement, the present disclosure relates to any one of the aforementioned methods, wherein the dissolution aid scaffold is a piece of porous material. In certain arrangements, the present disclosure relates to any one of the aforementioned methods, wherein the dissolution aid scaffold is a piece of porous material which is configured and adapted so that fluid passing through the bottom compartment and out the distal end of the ampoule passes through the piece of porous material and reconstitutes the polymerizable component adsorbed thereon.

In certain arrangements, the present disclosure relates to any one of the aforementioned methods, wherein the dissolution aid scaffold is a piece of plastic or glass. In certain arrangements, the present disclosure relates to any one of the aforementioned methods, wherein the dissolution aid scaffold is a piece of polyethylene (PE), polypropylene (PP), high-density polyethylene (HDPE), ultra-high molecular weight polyethylene (UHMWPE), polytetrafluoroethylene (PTFE), nylon, polyvinylidine fluoride (PVDF), polyethersulfone (PES), ethyl vinyl acetate (EVA), or a co-polymer thereof. In certain embodiments, the present invention relates to any one of the aforementioned methods, wherein the dissolution aid scaffold is a piece of polyester batting or other fibrous filter media material.

In certain arrangements, the present disclosure relates to any one of the aforementioned methods, wherein the dissolution aid scaffold is shaped like a disc and has a diameter equal to that of the ampoule.

In certain arrangements, the present disclosure relates to any one of the aforementioned methods, wherein the dissolution aid scaffold is at least about 6,35mm (0,25") from the distal end of the ampoule. In certain arrangements, the present disclosure relates to any one of the aforementioned methods, wherein the dissolution aid scaffold is at least about 12,7mm (0,5") from the distal end of the ampoule. In certain arrangements, the present disclosure relates to any one of the aforementioned methods, wherein the dissolution aid scaffold is at least about 19,05mm (0,75") from the distal end of the ampoule.

In certain arrangements, the present disclosure relates to any one of the aforementioned methods, wherein instead of a hydrophobic septum, a floating plunger head is used, wherein the floating plunger head is positioned between the proximal and distal ends of the ampoule, thereby creating a top compartment, bounded by the proximal end of the syringe and the floating plunger head, and a bottom compartment, bounded by the distal end of the syringe and the floating plunger head; wherein the floating plunger head contains a check valve which, when opened, connects the top compartment and the bottom compartment; the floating plunger head is configured and adapted to form a seal with the sidewall of the ampoule; and the floating plunger is axially translatable from a position near the proximal end of the ampoule to a position at the distal end of the ampoule.

In certain arrangements, the present disclosure relates to any one of the aforementioned methods, wherein instead of a hydrophobic septum, a floating plunger head is used, wherein the floating plunger head is positioned between the proximal and distal ends of the ampoule, thereby creating a top compartment, bounded by the proximal end of the syringe and the floating plunger head, and a bottom compartment, bounded by the distal end of the syringe and the floating plunger head; wherein the floating plunger head contains a frangible membrane which, when broken, connects the top compartment and the bottom compartment; the floating plunger head is configured and adapted to form a seal with the sidewall of the ampoule; and the floating plunger is axially translatable from a position near the proximal end of the ampoule to a position at the distal end of the ampoule.

Another aspect of the disclosure, not part of the present invention, relates to a method of preparing and applying a hydrogel to a surface; comprising the steps of:
(a) obtaining an applicator comprising:
   a syringe body having a sidewall, and an opening at each of its proximal and distal ends;
   a floating plunger head positioned between the proximal and distal ends of the syringe body, thereby creating a top compartment, bounded by the proximal end of the syringe and the floating plunger head, and a bottom compartment, bounded by the distal end of the syringe and the floating plunger head; wherein the floating plunger head contains a check valve which, when opened, connects the top compartment and the bottom compartment; the floating plunger head is configured and adapted to form a seal with the sidewall of the syringe body; and the floating plunger is axially translatable from a position near the proximal end of the syringe body to a position at the distal end of the syringe body;
   a plunger having a shaft, a distal end, a proximal end, and a plunger head provided at the distal end; the plunger inserted into the syringe body plunger head first through the proximal opening of the syringe body; the plunger head configured and adapted to form a seal when the plunger head engages the sidewall of the syringe body, wherein the plunger is axially translatable between a position where the plunger head is at the proximal end of the syringe body and a position wherein the plunger head is flush with the floating plunger at the distal end of the syringe body;
   a bladder having an opening at each of its proximal and distal ends;
   an adaptor which creates a fluid path from the distal end of the syringe body to the proximal end of the bladder;
   a tip having an opening at each of its proximal and distal ends, wherein the distal end of the bladder is connected to the proximal end of the tip;
   a liquid in the top compartment;
   a polymerizable component in the bottom compartment or in the adaptor; and
   a solid in the bladder;
(b) advancing the plunger head towards the floating plunger head, thereby causing the floating plunger head to move towards the distal end of the syringe body, causing the fluid in the bottom compartment to move through the adaptor into the bladder, thereby dissolving the polymerizable material;
(c) further advancing the plunger head towards the floating plunger head, thereby causing the check valve to open, allowing the fluid in the top compartment to pass through the bottom compartment, and through the adaptor, into the bladder, combining with the material in the bladder, thereby forming a pre-hydrogel mixture; and
(d) applying the liquid pre-hydrogel mixture to the surface via the tip, wherein the pre-hydrogel mixture gels to form a hydrogel on the surface.

In certain arrangements, the present disclosure relates to any one of the aforementioned methods, wherein the liquid pre-hydrogel mixture is applied by manually compressing the bladder to force the pre-hydrogel mixture out of the bladder, into the tip, and onto the surface.

In certain arrangements, the present disclosure relates to any one of the aforementioned methods, wherein the liquid pre-hydrogel mixture is applied by retracting the plunger back toward the proximal end of the syringe body and subsequently advancing the plunger to force the pre-hydrogel mixture out of the bladder, into the tip, and onto the surface.

In certain arrangements, the present disclosure relates to any one of the aforementioned methods, wherein the surface is dura mater. In certain arrangements, the present disclosure relates to any one of the aforementioned methods, wherein the surface is dura mater within the spinal column. In certain arrangements, the present disclosure relates to any one of the aforementioned methods, wherein the surface is cardiovascular tissue. In certain arrangements, the present disclosure relates to any one of the aforementioned methods, wherein the surface is gall tissue. In certain arrangements, the present disclosure relates to any one of the aforementioned methods, wherein the surface is urinary bladder tissue.

In certain arrangements, the present disclosure relates to any one of the aforementioned methods, further comprising the step of removing the syringe before applying the liquid pre-hydrogel mixture to the surface via the tip.

In certain arrangements, the present disclosure relates to any one of the aforementioned methods, wherein the liquid in the top compartment is water. In certain arrangements, the present disclosure relates to any one of the aforementioned methods, wherein the liquid in the top compartment is buffer. In certain arrangements, the present disclosure relates to any one of the aforementioned methods, wherein the liquid in the top compartment is an aqueous solution which comprises phosphates. In certain arrangements, the present disclosure relates to any one of the aforementioned methods, wherein the liquid in the top compartment is an aqueous solution which comprises borates.

In certain arrangements, the present disclosure relates to any one of the aforementioned methods, wherein the syringe body further comprises a liquid in the bottom compartment. In certain arrangements, the present disclosure relates to any one of the aforementioned methods, wherein the liquid in the bottom compartment is a concentrated salt solution, which when diluted forms a buffer. In certain arrangements, the present disclosure relates to any one of the aforementioned methods, wherein the liquid in the bottom compartment is an aqueous solution which comprises phosphates. In certain arrangements, the present disclosure relates to any one of the aforementioned methods, wherein the liquid in the bottom compartment is an aqueous solution which comprises borates.

In certain arrangements, the present disclosure relates to any one of the aforementioned methods, wherein the polymerizable material comprises a polyalkyleneimine. In certain arrangements, the present disclosure relates to any one of the aforementioned methods, wherein the polymerizable material comprises PEI.

In certain arrangements, the present disclosure relates to any one of the aforementioned methods, wherein the solid inside the bladder comprises an activated PEG. In certain arrangements, the present disclosure relates to any one of the aforementioned methods, wherein the solid inside the bladder comprises a PEG-(NHS)₂ reagent. In certain arrangements, the present disclosure relates to any one of the aforementioned methods, wherein the solid inside the bladder comprises PEG-SPA, PEG-SSeb, PEG-SG or a mixture thereof. In certain arrangements, the present disclosure relates to any one of the aforementioned methods, wherein the solid inside the bladder comprises PEG-SSeb. In certain arrangements, the present disclosure relates to any one of the aforementioned methods, wherein the solid inside the bladder comprises PEG-SPA.

In certain arrangements, the present disclosure relates to any one of the aforementioned methods, wherein the syringe body is tubular. In certain arrangements, the present disclosure relates to any one of the aforementioned methods, wherein the syringe body is plastic.

In certain arrangements, the present disclosure relates to any one of the aforementioned methods, wherein the length of the syringe body is between about 25,4mm (1") and about 127mm (5"). In certain arrangements, the present disclosure relates to any one of the aforementioned methods, wherein the length of the syringe body is between about 50,8mm (2") and about 101,6mm (4").

In certain arrangements, the present disclosure relates to any one of the aforementioned methods, wherein the length of the syringe body is about 76,2mm (3"). In certain arrangements, the present disclosure relates to any one of the aforementioned methods, wherein the length of the syringe body is about 50,8mm (2"). In certain embodiments, the present invention relates to any one of the aforementioned methods, wherein the length of the syringe body is about 25.4mm (1"). In certain arrangements, the present disclosure relates to any one of the aforementioned methods, wherein the average outer diameter of the syringe body is between about 2,54mm (0.1") and about 25,4mm (1"). In certain arrangements, the present disclosure relates to any one of the aforementioned methods, wherein the average outer diameter of the syringe body is between about 2,54mm (0.1") and about 10,16mm (0.4"). In certain arrangements, the present disclosure relates to any one of the aforementioned methods, wherein the average outer diameter of the syringe body is between about 10, 16mm (0.4") and about 15,24mm (0.6"). In certain arrangements, the present disclosure relates to any one of the aforementioned methods, wherein the average outer diameter of the syringe body is between about 15,24mm (0.6") and about 20,32mm (0.8"). In certain arrangements, the present disclosure relates to any one of the aforementioned methods, wherein the average outer diameter of the syringe body is between about 20.32mm (0.8") and about 25,4mm (1").In certain arrangements, the present disclosure relates to any one of the aforementioned methods, wherein the average outer diameter of the syringe body is about 6,604mm (0.26"). In certain arrangements, the present disclosure relates to any one of the aforementioned methods, wherein the average outer diameter of the syringe body is about 9,906mm (0.39"). In certain arrangements, the present disclosure relates to any one of the aforementioned methods wherein the average outer diameter of the syringe body is about 13.97mm (0.55"). In certain arrangements, the present disclosure relates to any one of the aforementioned methods, wherein the average outer diameter of the syringe body is about 24.638mm (0.97").

In certain embodiments, the present invention relates to any one of the aforementioned syringes, further comprising a dissolution aid scaffold; wherein the polymerizable component is adsorbed onto the dissolution aid scaffold. In certain embodiment, the present invention relates to any one of the aforementioned syringes, wherein the dissolution aid scaffold is a piece of porous material. In certain embodiment, the present invention relates to any one of the aforementioned syringes, wherein the dissolution aid scaffold is a piece of porous material which is configured and adapted so that fluid passing through the bottom compartment and out the distal end of the syringe body passes through the piece of porous material and reconstitutes the polymerizable component adsorbed thereon.

In certain embodiments, the present invention relates to any one of the aforementioned syringes, wherein the dissolution aid scaffold is a piece of plastic or glass. In certain embodiments, the present invention relates to any one of the aforementioned syringes, wherein the dissolution aid scaffold is a piece of polyethylene (PE), polypropylene (PP), high-density polyethylene (HDPE), ultra-high molecular weight polyethylene (UHMWPE), polytetrafluoroethylene (PTFE), nylon, polyvinylidine fluoride (PVDF), polyethersulfone (PES), ethyl vinyl acetate (EVA), or a co-polymer thereof. In certain embodiments, the present invention relates to any one of the aforementioned syringes, wherein the dissolution aid scaffold is a piece of polyester batting or other fibrous filter media material.

In certain embodiments, the present invention relates to any one of the aforementioned syringes, wherein the dissolution aid scaffold is shaped like a disc and has a diameter equal to that of the syringe body.

In certain embodiments, the present invention relates to any one of the aforementioned syringes, wherein the dissolution aid scaffold is shaped like a disc and has a diameter equal to that of the adaptor.

In certain arrangements, the present disclosure relates to any one of the aforementioned methods, wherein the floating plunger is at least about 6.35mm (0.25") from the proximal end of the syringe body. In certain arrangements, the present disclosure relates to any one of the aforementioned methods, wherein the floating plunger is at least about 12,7mm (0,50") from the proximal end of the syringe body. In certain arrangements, the present disclosure relates to any one of the aforementioned methods, wherein the floating plunger is at least about 19.05mm (0,75") from the proximal end of the syringe body.

In certain arrangements, the present disclosure relates to any one of the aforementioned methods, wherein the check valve is pressure sensitive. In certain arrangements, the present disclosure relates to any one of the aforementioned methods, wherein the check valve is a ball check valve, a diaphragm check valve, a swing check valve, a lift-check valve, a double check valve, a double ball check valves, a piston check valve, a wafer check valve, a duckbill check valve, an umbrella check valve, or a ball-and-cone check valve.

In certain arrangements, the present disclosure relates to any one of the aforementioned methods, wherein instead of a check valve, the applicator has a bypass built into the outer part of the syringe body, thereby accomplishing the same function. In certain arrangements, the present disclosure relates to any one of the aforementioned methods, wherein instead of a check valve, the floating plunger has frangible membrane, which when broken allows for fluid communication between the top and bottom compartments, thereby accomplishing the same function.

In certain arrangements, the present disclosure relates to any one of the aforementioned methods, wherein the bladder is tubular.

In certain arrangements, the present disclosure relates to any one of the aforementioned methods, wherein the bladder is made from a compressible material, wherein the compressible material permits manual pressurization. In certain arrangements, the present disclosure relates to any one of the aforementioned methods, wherein the bladder is plastic.

In certain arrangements, the present disclosure relates to any one of the aforementioned methods, wherein the length of the bladder is between about 25,4mm (1") and about 127mm (5"). In certain arrangements, the present disclosure relates to any one of the aforementioned methods, wherein the length of the bladder is between about 50,8mm (2") and about 101,6mm (4"). In certain arrangements, the present disclosure relates to any one of the aforementioned methods, wherein the length of the bladder is about 76,2mm (3"). In certain arrangements, the present disclosure relates to any one of the aforementioned methods, wherein the length of the bladder is about 50,8mm (2").

In certain embodiments, the present invention relates to any one of the aforementioned methods, wherein the length of the bladder is about 25.4mm (1").

In certain arrangements, the present disclosure relates to any one of the aforementioned methods, wherein the average outer diameter of the bladder is between about 2,54mm (0.1") and about 25,4mm (1"). In certain arrangements, the present disclosure relates to any one of the aforementioned methods, wherein the average outer diameter of the bladder is between about 2,54mm (0.1") and about 7,62mm (0.3"). In certain arrangements, the present disclosure relates to any one of the aforementioned methods, wherein the average outer diameter of the bladder is between about 7,62mm (0.3") and about 12,7mm (0.5"). In certain arrangements, the present disclosure relates to any one of the aforementioned methods, wherein the average outer diameter of the bladder is between about 12,7mm (0.5") and about 17,78mm (0.7"). In certain arrangements, the present disclosure relates to any one of the aforementioned methods, wherein the average outer diameter of the bladder is between about 17,78mm (0.7") and about 25,4mm (1").

In certain arrangements, the present disclosure relates to any one of the aforementioned methods, wherein the adaptor is tubular. In certain arrangements, the present disclosure relates to any one of the aforementioned methods, wherein the adaptor is plastic.

In certain arrangements, the present disclosure relates to any one of the aforementioned methods, wherein the average wall thickness of the bladder is between about 0,0254mm (0.001") and about 12,7mm (0.5"). In certain arrangements, the present disclosure relates to any one of the aforementioned methods, wherein the average wall thickness of the bladder is between about 0,0254mm (0.001") and about 0,254mm (0.01"). In certain arrangements, the present disclosure relates to any one of the aforementioned applicators, wherein the average wall thickness of the bladder is between about 0,254mm (0.01") and about 2,54mm (0.1"). In certain arrangements, the present disclosure relates to any one of the aforementioned methods, wherein the average wall thickness of the bladder is between about 2,54mm (0.1") and about 12,7mm (0.5").

In certain arrangements, the present disclosure relates to any one of the aforementioned methods, wherein the length of the adaptor is between about 2,54mm (0.1") and about 50,8mm (2"). In certain arrangements, the present disclosure relates to any one of the aforementioned applicators, wherein the length of the adaptor is between about 2.54mm (0.1") and about 12,7mm (0.5"). In certain arrangements, the present disclosure relates to any one of the aforementioned methods wherein the length of the adaptor is between about 12,7mm (0.5") and about 25,4mm (1"). In certain arrangements,

the present disclosure relates to any one of the aforementioned methods, wherein the length of the adaptor is between about 25,4mm (1") and about 38,1mm (1.5"). In certain arrangements, the present disclosure relates to any one of the aforementioned methods, wherein the length of the adaptor is between about 38,1mm (1.5") and 50,8mm (2").

In certain arrangements, the present disclosure relates to any one of the aforementioned methods, further comprising a removable separator or valve at the distal end of the syringe body that controls fluid flow from syringe body to the adaptor. In certain arrangements, the present disclosure relates to any one of the aforementioned methods, wherein the tip is a cannula tip, a bent cannula tip, a bayonet¬ style cannula tip, a brush applicator tip, a duck-bill tip, a textile mitt, a spray tip, a foam swab, or a silicon tube. In certain arrangements, the present disclosure relates to any one of the aforementioned methods, wherein the tip is a bent cannula tip or a bayonet-style cannula tip.

In certain arrangements, the present disclosure relates to any one of the aforementioned methods, wherein the length of the tip is between about 2,54mm (0.1") and about 254mm (10"). In certain arrangements, the present disclosure relates to any one of the aforementioned methods, wherein the length of the tip is between about 25,4mm (1") and about 50,8mm (2"). In certain arrangements, the present disclosure relates to any one of the aforementioned methods, wherein the length of the tip is between about 50,8mm (2") and about 76,2mm (3"). In certain arrangements, the present disclosure relates to any one of the aforementioned methods, wherein the length of the tip is between about 76,2mm (3") and about 101,6mm (4"). In certain arrangements, the present disclosure relates to any one of the aforementioned methods, wherein the length of the tip is between about 101,6,, (4") and about 127mm (5"). In certain arrangements, the present disclosure relates to any one of the aforementioned methods, wherein the length of the tip is between about 127mm (5") and about 152,4mm (6"). In certain arrangements, the present disclosure relates to any one of the aforementioned methods, wherein the length of the tip is between about 152,4mm (6") and about 177,8mm (7"). In certain arrangements, the present disclosure relates to any one of the aforementioned methods, wherein the length of the tip is between about 177,8mm (7") and about 203,2mm (8"). In certain arrangements, the present disclosure relates to any one of the aforementioned methods, wherein the length of the tip is between about 203,2mm (8") and about 228,6mm (9"). In certain arrangements, the present disclosure relates to any one of the aforementioned methods, wherein the length of the tip is between about 228,6mm (9") and about 254mm(10").

In certain arrangements, the present disclosure relates to any one of the aforementioned methods, wherein the diameter of the distal end of the tip is between about 0,381mm (0.015") and about 1,397mm (0.55"). In certain arrangements, the present disclosure relates to any one of the aforementioned methods, wherein the diameter of the distal end of the tip is between about 0,381mm (0.015") and about 5,08mm (0.2"). In certain arrangements, the present disclosure relates to any one of the aforementioned methods, wherein the diameter of the distal end of the tip is between about 0,889mm (0.035") and 3,81mm (0.15"). In certain arrangements, the present disclosure relates to any one of the aforementioned methods, wherein the diameter of the distal end of the tip is between about 0,1397mm (0.055") and 2,54mm (0.1"). In certain arrangements, the present disclosure relates to any one of the aforementioned methods, wherein the diameter of the distal end of the tip is about 2,032mm (0.08").

In certain embodiments, the present invention relates to any one of the aforementioned methods, further comprising a screen or filter placed in the tip.

### STERILIZATION PROCEDURES

A variety of procedures the applicators and/or the chemical composition contained therein. Sterilization may be accomplished by chemical, physical, or irradiation techniques. Examples of chemical methods include exposure to ethylene oxide or hydrogen peroxide vapor. Examples of physical methods include sterilization by heat (dry or moist), retort canning, and filtration. The British Pharmacopoeia recommends heating at a minimum of 160 °C for not less than 2 hours, a minimum of 170 °C for not less than 1 hour and a minimum of 180 °C for not less than 30 minutes for effective sterilization. For examples of heat sterilization, see U.S. Patent 6,136,326. Passing the chemical composition through a membrane can be used to sterilize a composition. For example, the composition is filtered through a small pore filter such as a 0.22 micron filter which comprises material inert to the composition being filtered. In certain instances, the filtration is conducted in a Class 100,000 or better clean room. Examples of irradiation methods include gamma irradiation, electron beam irradiation, microwave irradiation, and irradiation using visible light. One preferred method is electron beam irradiation, as described in U.S. Patents 6,743,858; 6,248,800; and 6,143,805.

There are several sources for electron beam irradiation. The two main groups of electron beam accelerators are: (1) a Dynamitron, which uses an insulated core transformer, and (2) radio frequency (RF) linear accelerators (linacs). The Dynamitron is a particle accelerator (4.5 MeV) designed to impart energy to electrons. The high energy electrons are generated and accelerated by the electrostatic fields of the accelerator electrodes arranged within the length of the glass-insulated beam tube (acceleration tube). These electrons, traveling through an extension of the evacuation beam tube and beam transport (drift pipe) are subjected to a magnet deflection system in order to produce a "canned" beam, prior to leaving the vacuum enclosure through a beam window. The dose can be adjusted with the control of the percent scan, the beam current, and the conveyor speed. In certain instances, the electron-beam radiation employed may be maintained at an initial fluence of at least about 2 µCurie/cm², at least about 5 µCurie/cm², at least about 8 µCurie/cm², or at least about 10 µCurie/cm². In certain instances, the electron-beam radiation employed has an initial fluence of from about 2 to about 25 µCurie/cm². In certain instances, the electron-beam dosage is from about 5 to 50 kGray, or from about 15 to about 20 kGray with the specific dosage being selected relative to the density of material being subjected to electron-beam radiation as well as the amount of bioburden estimated to be therein. Such factors are well within the skill of the art.

The applicators and/or compositions to be sterilized may be in any type of at least partially electron beam permeable container such as glass or plastic. The penetration of electron beam irradiation is a function of the packaging. If there is not enough penetration from the side of a stationary electron beam, the container may be flipped or rotated to achieve adequate penetration. Alternatively, the electron beam source can be moved about a stationary package. In order to determine the dose distribution and dose penetration in product load, a dose map can be performed. This will identify the minimum and maximum dose zone within a product.

Procedures for sterilization using visible light are described in U.S. Patent 6,579,916. The visible light for sterilization can be generated using any conventional generator of sufficient power and breadth of wavelength to effect sterilization. Generators are commercially available under the tradename PureBright^{®} in-line sterilization systems from PurePulse Technologies, Inc. 4241 Ponderosa Ave, San Diego, Calif. 92123, USA. The PureBright^{®} in-line sterilization system employs visible light to sterilize clear liquids at an intensity approximately 90000 times greater than surface sunlight. If the amount of UV light penetration is of concern, conventional UV absorbing materials can be used to filter out the UV light.

In a preferred embodiment, the composition in the applicator or portion thereof is sterilized to provide a Sterility Assurance Level (SAL) of at least about 10⁻³. The Sterility Assurance Level measurement standard is described, for example, in ISO/CD 1493 7. In certain embodiments, the Sterility Assurance Level may be at least about 10⁻⁴, at least about 10⁻⁵, or at least about 10⁻⁶

As discussed above, in certain arrangements one or more of the compositions, reagents, or components of a kit has been sterilized. The sterilization may be achieved using gamma radiation, e-beam radiation, dry heat sterilization, ethylene oxide sterilization, or a combination of any of them. The compositions, reagents, or components of the kits can be sterilized in an aqueous solution or neat.

In certain embodiments a compound present in an applicator or portion thereof (as described herein) has been sterilized by e-beam radiation between 2-40 kGy; or between 3-20 kGy; or between 5-12 kGy.

In certain embodiments, said sterilization is carried out below 30 °C. In certain embodiments, said sterilization is carried out below 20 °C. In certain embodiments, said sterilization is carried out below 10 °C. In certain embodiments, said sterilization is carried out below 0 °C.

### KITS

In another aspect not part of the present invention kits are provided containing one or more applicators of the invention. A "kit," as used herein, typically defines a package or an assembly including one or more of the applicators of the invention, and/or other compositions associated with the invention, for example, as described herein. Each of the compositions of the kit may be provided in liquid form (e.g., in solution), or in solid form (e.g., a dried powder). In certain cases, some of the compositions may be constitutable or otherwise processable (e.g., to an active form), for example, by the addition of a suitable solvent or other species, which may or may not be provided with the kit. Examples of other compositions or components associated with the invention include, but are not limited to, solvents, surfactants, diluents, salts, buffers, emulsifiers, chelating agents, fillers, antioxidants, binding agents, bulking agents, preservatives, drying agents, antimicrobials, needles, syringes, packaging materials, tubes, bottles, flasks, beakers, dishes, frits, filters, rings, clamps, wraps, patches, containers, and the like, for example, for using, modifying, assembling, storing, packaging, preparing, mixing, diluting, and/or preserving the compositions components for a particular use.

A kit may include instructions in any form that are provided in connection with the applicators of the invention in such a manner that one of ordinary skill in the art would recognize that the instructions are to be associated with the applicator of the invention. For instance, the instructions may relate to the use, modification, mixing, diluting, preserving, assembly, storage, packaging, and/or preparation of the applicators and/or other compositions associated with the kit. In some cases, the instructions may also include instructions for the use of the applicators. The instructions may be provided in any form recognizable by a user as a suitable vehicle for containing such instructions; for example, written or published, verbal, audible (e.g., telephonic), digital, optical, visual (e.g., videotape, DVD, etc.) or electronic communications (including Internet or web-based communications), provided in any manner.

In certain embodiments, different parts of the applicators may be packaged separately (e.g., in Mylar pouches). For example, in some circumstances it may be advantageous to package the syringe body (with plunger and caped at the distal end) and the bladder (caped at the proximal end and with a tip or a cap at the distal end) separately.

### DEFINITIONS

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03.

The term "bladder" as used herein refers to a hollow structure which has an open distal end and an open proximal end. A flexible tube is an example of a bladder; as is a PVC plastisol bladder.

The term "check valve" as used herein refers to a mechanical device, a valve, which normally allows fluid to flow through it in only one direction.

The term "fluid bypass" as used herein refers to a structural aspect of, for example, a syringe body, that allows fluid to flow from one compartment to another once a plunger head, or the like, is distally advanced. See, for example, US Patent No. 4,735,616, which describes a twin bypass syringe.

The term "tip" as used herein is known to those skilled in the art and refers to a mechanical device designed to control the characteristics of a fluid flow as it exits from an enclosed chamber (such as an applicator body) into some medium. A tip is often a tube of varying diameter, and it can be used to direct or modify the flow of a liquid or gas. Tips are frequently used to control the rate of flow, speed, direction, and/or the pressure of the stream that emerges from them. In certain embodiments the proximal end of a tip, wherein the fluid flow enters, will have a larger diameter than the distal end of a tip, where the fluid flow exists. This is known as a convergent tip (i.e., narrowing down from a wide diameter to a smaller diameter in the direction of the flow). In other embodiments the tip can be characterized as divergent (i.e., expanding from a smaller diameter to a larger one).

The term "brush" or "brush cannula" as used herein is known to those skilled in the art. The name represents the function of the brush: It is constructed to enable liquid to flow through the bristles for an application. The brushes can be attached to a wide variety of media that dispense liquid, and can be made out of many types of bristle material and configurations. In certain embodiments herein the brush cannula is connected to an applicator body. Brush cannulas are also known as flow-thru brushes; the terms are used interchangeably herein.

The term "activated PEG" as used herein is known to those skilled in the art and refers to poly(ethylene) glycols (linear or branched) which have at least one end activated for conjugation with other molecules. Shown below are chemical structures for polyethylene glycol (PEG), mono-methylated polyethylene glycol (mPEG), an activated mPEG and a bis-activated PEG.

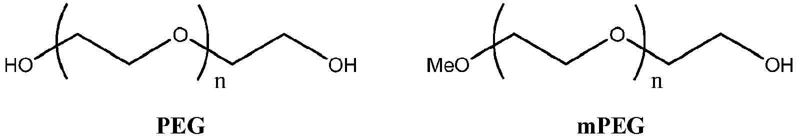

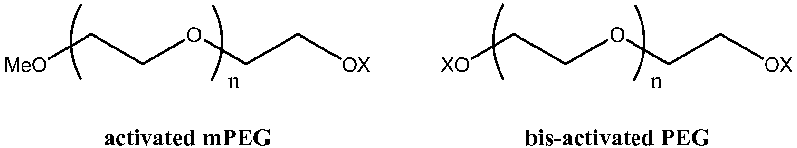

In the structures provided above n is a positive integer. In a batch of activated PEG different individual molecules will have a different values of n (i.e., the mixture is polydisperse); these mixtures are often characterized by an average molecular weight, which can be converted into an average value for n. In certain embodiments herein, the average n is between about 10 and about 200. In other embodiments the average n is between about 80 and about 120. In yet other embodiments, the average n is about 100. In the structures provided above, X can comprise a variety of chemical moieties such as, for example, a *N-*succinimide, a *N*-maleimide, a nitro, an aldehyde, an amine, a thiol, a ketal, an acetal, or a carbonate. In certain embodiments, X is selected from the group consisting of -CH₂C(=O)O(*N*-succinimidyl), -(CH₂)₂C(=O)O(*N*-succinimidyl), -(CH₂)₃C(=O)O(*N*-succinimidyl) ["PEG-SPA"], -(CH₂)₄C(=O)O(*N*-succinimidyl), -(CH₂)₅C(=O)O(*N*-succinimidyl), -(CH₂)₆C(=O)O(*N*-succinimidyl), -(CH₂)₇C(=O)O(*N*-succinimidyl), -(CH₂)₈C(=O)O(*N*-succinimidyl), -(CH₂)₉C(=O)O(*N-*succinimidyl), -C(=O)CH₂C(=O)O(*N*-succinimidyl), -C(=O)(CH₂)₂C(=O)O(*N*-succinimidyl), -C(=O)(CH₂)₃C(=O)O(*N*-succinimidyl) ["PEG-SG"], -C(=O)(CH₂)₄C(=O)O(*N*-succinimidyl) ["PEG-SA"], -C(=O)(CH₂)₅C(=O)O(*N*-succinimidyl), -C(=O)(CH₂)₆C(=O)O(*N*-succinimidyl), -C(=O)(CH₂)₇C(=O)O(*N*-succinimidyl), -C(=O)(CH₂)₈C(=O)O(*N*-succinimidyl) ["PEG-SSeb"], -C(-O)(CH₂)₉C(-O)O(*N*-succinimidyl), -C(=O)(*p*-nitrophenyl), -CH₂CH₂C(-O)H, -CH₂CH₂CH₂NH₂, -CH₂CH₂CH(OCH₂CH₃)₂, -CH₂CH₂SH, -CH₂CH₂CH₂N(H)C(=O)CH₂CH₂(*N*-maleimidyl), and -O(C=O)O(*p*-nitrophenyl).

In certain embodiments, the bulk density of the activated PEG is between about 0.1 g/cc and 0.2 g/cc. In certain embodiments, the bulk density of the activated PEG is between about 0.2 g/cc and 0.3 g/cc. In certain embodiments, the bulk density of the activated PEG is between about 0.3 g/cc and 0.4 g/cc. In certain embodiments, the bulk density of the activated PEG is between about 0.4 g/cc and 0.5 g/cc. In certain embodiments, the bulk density of the activated PEG is between about 0.5 g/cc and 0.6 g/cc. In certain embodiments, the bulk density of the activated PEG is between about 0.7 g/cc and 0.8 g/cc. In certain embodiments, the bulk density of the activated PEG is between about 0.9 g/cc and 1 g/cc. In certain embodiments, the bulk density of the activated PEG is between about 1 g/cc and 10 g/cc. Methods of preparing activated PEG with a bulk density of greater than about 0.25 g/cc is provided in Example 7 below.

The term "PEG(NHS)₂" refers to a polyethylene glycol having -C(=O)O((N-succinimidyl) at both ends of the polymer chain. PEG(NHS)₂ can be prepared in variety of ways, such as by using either of the following methods. In method 1, a polyethylene glycol is subjected to oxidative conditions in order to oxidize the two termini to the corresponding carboxylic acids [HO₂CCH₂O-PEG-OCH₂CO₂H], followed by transformation to the bis(NHS ester). In method 2, PEG(NHS)₂ is prepared by alkylation of the two termini of a polyethylene glycol with acrylonitrile to give NCCH₂CH₂O-PEG-OCH₂CH₂CN, followed by hydrolysis to the bis(acid) [HO₂CCH₂CH₂O-PEG-OCH₂CH₂CO₂H], and then transformation to the bis(NHS ester).

As used here, "PEG-SPA" refers to the following structure: 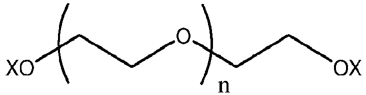 wherein X is -(CH₂)₃C(=O)O(*N*-succinimidyl); and n is an integer (e.g., from 10 to 200).

As used herein, "PEG-SG" refers to the following structure: 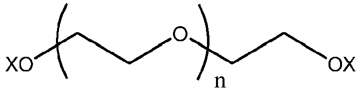 wherein X is -C(=O)(CH₂)₃C(=O)O(*N*-succinimidyl); and n is an integer (e.g., from 10 to 200).

As used herein, "PEG-SA" refers to the following structure: 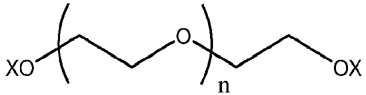 wherein X is -C(=O)(CH₂)₄C(=O)O(*N*-succinimidyl); and n is an integer (e.g., from 10 to 200).

As used herein, "PEG-SSeb" refers to the following structure: 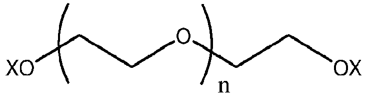 wherein X is -C(=O)(CH₂)₈C(=O)O(*N*-succinimidyl); and n is an integer (e.g., from 10 to 200).

As used herein, "plastic" refers to polyacrylics, silicones, polyolefins, polystyrenes, polyesters, polyethers, polyurethanes, polycarbonates, polyamines, or co-polymers thereof.

As used herein, "silicones" (polymerized siloxanes or polysiloxanes) are mixed inorganic-organic polymers with the chemical formula [R₂SiO]ₙ, where R may be an organic group such as methyl, ethyl, and phenyl. These materials consist of an inorganic silicon-oxygen backbone with organic side groups attached to the silicon atoms, which are four-coordinate. In some cases organic side groups can be used to link two or more of these backbones together. By varying the -Si-O- chain lengths, side groups, and crosslinking, silicones can be synthesized with a wide variety of properties and compositions.

In preparing hydrogels, the chemical reaction that causes the liquid formulation to gel may be dependent upon pH. For example, when forming a PEI/PEG-SG hydrogel, the PEI solution is basic and the buffer solution is acidic such that when mixed together, the resulting liquid has the correct pH to cause the dissolve PEG-SG to polymerize and gel within a reasonable time. Therefore, the term "buffer," as used herein, refers to a buffer that allow the formation of a hydrogel.

As used herein "adsorbed" means that a film of molecules (the adsorbate) is formed on a substrate. As used herein, one example of a substrate onto which a solid or viscous liquid is adsorbed is a dissolution aid scaffold.

As used here "dissolution aid scaffold" refers to a solid material, such as a plastic or glass, which has a surface area onto which a solid or viscous liquid, or the like, can be deposited. As the term suggests, a material deposited on the scaffold is easier to dissolve in a liquid than the bulk material (because, in part, due to the higher surface area the material presents when spread over the scaffold).

As used herein, the term "septum" refers to a partition separating two cavities or spaces, wherein the partition is permeable to liquids under certain conditions (such as in increase in pressure). A hydrophobic membrane is an example of a septum, as the term is used herein.

As used herein, the term "patient" refers to any animal in need, including primates, in particular humans, and other mammals such as equines, cattle, swine and sheep; and poultry and pets in general.

As used here, a seal which is "frangible" is one which is capable of being broken (e.g., by puncture).

### EXEMPLIFICATION

The invention now being generally described, it will be more readily understood by reference to the following examples which are included merely for purposes of illustration of certain aspects and embodiments of the present invention, and are not intended to limit the invention.

### Example 1

A hydrophilic filter (e.g., Filtrona D3602C or Filtrona D3737B; 12.25 mm diameter x 5 mm length), was placed in a 5 mL BD syringe. A 100 mL volumetric flask was used to prepared a solution of PEI PR8515 (5.0012 g) filled to volume with MeOH. Of the PEI solution thus prepared, 254 µl was deposited onto the hydrophilic fliter. The methanol was then removed under reduced pressure. A hydrophobic filter (e.g., Filtrona D3602A, D3602B, D3602C, or D4017) was then inserted in the proximal end of the syringe. A buffer salt solution was then prepared by dissolving sodium tetraborate decahydrate (3.3371 g), and NaH₂PO₄ (2.6987 g) in a 250 mL volumetric flask; one drop of food coloring was added to the volumetric to act as a visualizing aid. To the proximal end of the syringe, 2.40 mL of the buffer solution was dispensed. The plunger was inserted in the proximal end such that the buffer solution was not forced through the hydrophobic filter, and a cap placed on the luer fitting. The PEI on the dissolution aid scaffold (i.e., the hydrophilic filter) was then dissolved by depressing the plunger and forcing the solution first through the hydrophobic filter and then through the PEI embedded hydrophilic filter. The plunger was released to allow the pressure which had developed to equilibrate, and push the solution back toward the proximal end of the syringe. The solution was exchanged repeatedly for a total of at least ten cycles. The reconstituted PEI solution was then mixed with PEG-SSeb 3.4K (200 mg). The resulting gels set in an average of 30 seconds, and had a 24 hr equilibrium gravimetric swelling of 5% in pH 7.4 PBS at 37C. Samples were prepared for all 8 combinations of the listed filter types.

### Example 2

To a hydrophobic filter (Filtrona D3602B) inside a 5 mL BD syringe 254µL of the PEI/MEOH solution used in Example 1 was dispensed. The MeOH was removed under reduced pressure. The Buffer solution used in Example 1 was then dispensed on top of the treated filter and was observed to absorb into the filter allowing dissolution of the PEI. Thus, the hydrophobic filter acted as a hydrophilic filter once PEI was deposited on to the filter.

### Example 3

An applicator similar to the one depicted in Figure 1 was prepared by placing 200 mg of PEG-SSeb within a silicone bladder with a long applicator tip on one end and a luer lock tube adapter with cap on the other end. This portion of the device was sealed within one compartment of a two compartment peelable foil pouch. A PEI solution was prepared to reconstitute the PEG-SSeb at time of use by dispensing 1.88 mL of a buffered solution containing approximately 2.68 mM polyethyleneimine Mw = 2000, approximately 58.2 mM NaH₂PO₄, approximately 38.8 mM Na₂HPO₄, and approximately 35.0 mM Na₂B₄O₇•10H₂O into a 3 mL syringe. The syringe was sealed within the second compartment of the two compartment peelable foil pouch. The applicators within foil pouches were exposed to approximately 20 - 25 kGy of e-beam radiation. At time of use the in situ polymerizing test article was prepared by opening the two compartments of the foil pouch. The test article was assembled by connecting the squeezable fitment with applicator tube adapter to a 3 mL syringe containing the reconstitution buffer through the luer lock fitting. The reconstitution solution was dispensed into the squeezable bladder, the PEG3400-SSeb active component was dissolved by shaking the device for at least five seconds, and the resultant solution was expressed through the applicator tube onto the target area. Gelation of the dispensed material occurred within approximately 30 seconds.

### Example 4

An applicator similar to the one depicted in Figure 1 was prepared by placing 200 mg of PEG-SSeb within the silicone bladder with a long applicator tip on one end and a modified luer lock tube adapter with cap on the other end. The luer lock tube adapter was modified by inserting two hydrophilic filters into the adapter, adding 120 µL of a PEI methanol solution (made by dissolving 10.1152 g of PEI Mw = 2000 in methanol and filling the 100 mL volumetric to volume), and then removing the methanol under vacuum. This portion of the device was sealed within one compartment of a two compartment peelable foil pouch. A reconstitution solution was prepared to reconstitute the PEI followed by the PEG-SSeb at time of use by dispensing 2.08 mL of a buffered solution containing approximately 87.3 mM NaH₂PO₄, approximately 9.7 mM Na₂HPO₄, and approximately 35.0 mM Na₂B₄O₇•10H₂O into a 3 mL syringe. The syringe was sealed within the second compartment of the two compartment peelable foil pouch. The applicators within foil pouches were exposed to approximately 20 - 25 kGy of e-beam radiation. At time of use the in situ polymerizing test article was prepared by opening the two compartments of the foil pouch. The test article was assembled by connecting the squeezable fitment with applicator tube adapter to a 3 mL syringe containing the reconstitution buffer through the luer lock fitting. The site of application was blotted dry. The reconstitution solution was slowly dispensed through the PEI loaded luer lock tube adapter and into the squeezable bladder, the PEG3400-SSeb active component was dissolved by shaking the device for at least five seconds, and the resultant solution was expressed through the applicator tube onto the target area. Gelation of the dispensed material occurred within approximately 30 seconds.

### Example 5

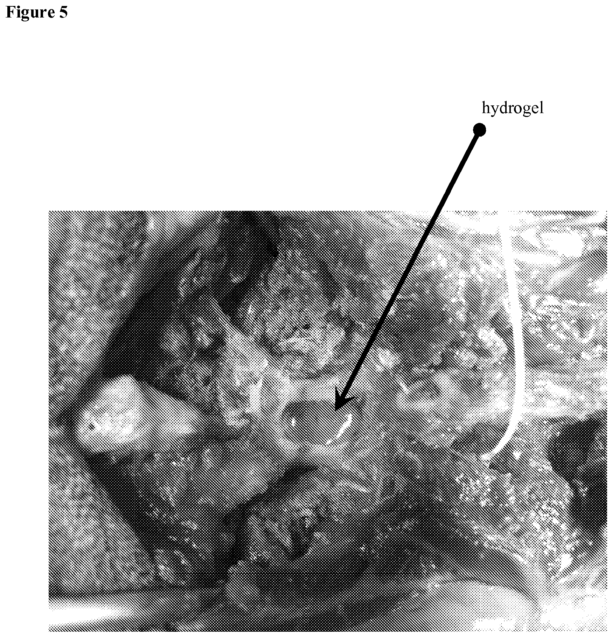

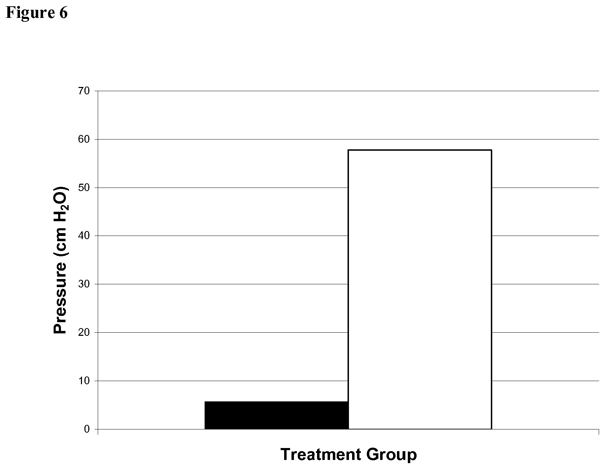

The applicator from Example 4 was used in a pre-clinical canine lumbar durotomy repair evaluation. Under inhalant anesthesia, a midline incision was made over the lumbar spine and the skin and musculature reflected. A hemilaminectomy was made in the L3 vertebra using a burr drill bit. A 0.9 mm x 0.5 mm polyurethane catheter was inserted into the subarachnoid space for baseline CSF pressure readings. The catheter was connected to an intracranial pressure (ICP) transducer and three way stop cock. Once implanted, a baseline CSF pressure was recorded. After the baseline reading, additional musculature was reflected to expose the dorsal process and lamina of the L2 and L5 vertebra. The dorsal aspect of both vertebral lamina were removed (including the dorsal process) using a burr drill bit, and the dura exposed. Following exposure of the dura, an approximate 1.0 cm incision was made through the dura and arachnoid, and the incision approximated with 6-0 monofilament nylon suture. Following this incision, the CSF freely egressed from the incision. The incision was blotted dry and an in situ polymerizing solution was applied via the described applicator to a depth of approximately 1 mm over midline in the six (n=6) treated animals (see Figure 5). In six (n=6) dogs, the durotomy site was closed with sutures only. Once the test article had significantly cured or following sutured closure, the durotomy site was tested for CSF leakage with a saline infusion via the three way stop cock. The ICP transducer was monitored during saline infusion until a pressure of at least 41 cm of H₂O was reached and maintained for approximately 30 seconds. The L2 and L5 durotomy sites for each of the treated sites remained leak free at an average pressure of 58 cm of H₂O, which was maintained for at least 30 seconds. The L2 and L5 durotomy sites for each of the control sites leaked at an average pressure of 6 cm of H₂O, none of the control sites were able to maintain a pressure close to 41 cm of H₂O, especially for 30 seconds. Results are shown in Figure 6. Once pressure testing was completed, the skin and musculature were closed and tissue adhesive applied.

At four to five days post surgery, the animals were evaluated for post surgical CSF leaks by MRI. During this evaluation, all 12 of the treated sites remained CSF leak free (100% leak free) while all 12 of the control sites displayed signs of a CSF leak (0% leak free).

The animals will continue to be evaluated for neurological deficits and clinical signs until the planned necropsy at either 2 or 4 months.

### Example 6

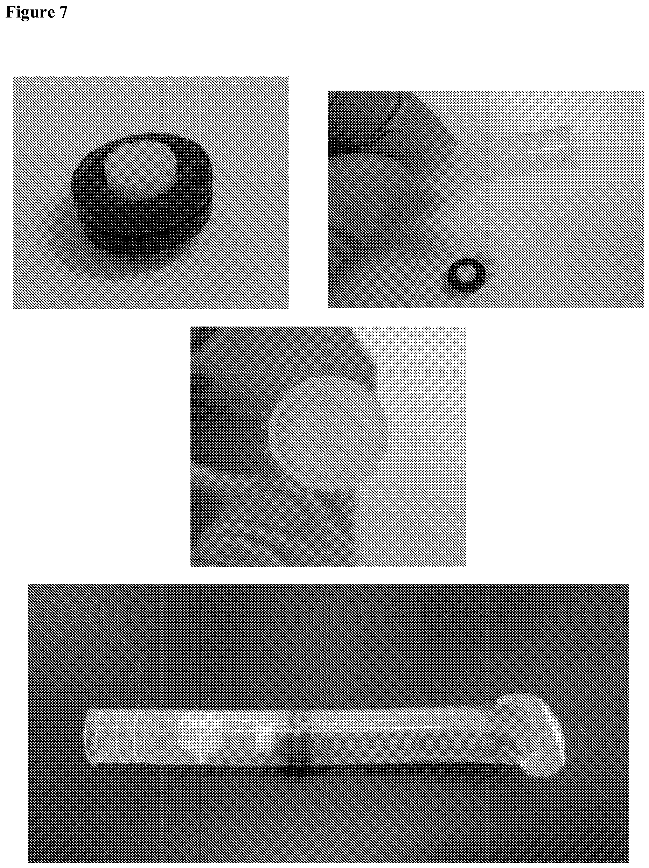

As noted above, certain arrangements not part of the invention do not utilize a syringe body. For example, one embodiment was prepared by inserting a septum comprised of a 3 mL plunger head with a hydrophobic porous filter in the middle inside a James Alexander Company Plastic amp with a friable seal at the distal end. PEI was placed in the compartment in the distal end of the amp (on a dissolution aid scaffold). A buffer solution was placed on the proximal side of the septum and the tube was sealed. See Figure 7.

At time of use the solution would be expressed into the front compartment to reconstitute the PEI. The tube would then be connected to the PEG bladder and the friable seal broken to combine the PEG powder and the PEI solution.

### Example 7

Melt processing of activated PEG, along with some further mechanical processing of the resulting product, are described below.
1. Activated PEG was placed into a glass vial and heated to 80° C for 30 minutes in order to fully melt the powder. The resulting liquid was placed into the syringe of a repeating pippetter and placed in 50 mL aloquates onto a dry lab bench. Once cooled, 1 gram of activated PEG beads were placed into a syringe and reconstituted. The activated PEG beads were dissolved within approximately 10 minutes and were found to be free from substantial amounts of air or foaminess.
2. Beads as described immediately above were subjected to an additional reduction in particle size by cutting with a razor blade. The resulting material was a coarse powder or somewhat varying particle size. One gram of the resulting material was placed into a syringe and reconstituted. The activated PEG particles were substantially dissolved within 5 minutes and were found to be free from substantial amounts of air or foaminess.
3. Activated PEG powder was placed into a glass vial and heated to 80° C for 30 minutes in order to fully melt the powder. The resulting liquid was placed into a 3 cc syringe. The 3 cc syringe was placed onto an air assisted sprayer and hooked up to a source of compressed air. The melted PEG was then pressurized and thus introduced into the air stream of the sprayer. The resulting spray of molten PEG occurred approximately 5 feet above the surface of a clean lab bench. The resulting material was removed from the surface of the lab bench resulting in an extremely fine powder. This powder had a bulk density of approximately 0.5 g/cc. 1 gram of this powder was placed into a 5 cc syringe and reconstituted. The reconstituted PEG having a volume of approximately 3 mL was found to have a compressability of approximately 0.2 cc due to dissolved or entrapped air. This was compared to a similar syringe of 1 gram of 0.25 g/cc bulk density powder formed by recrytallization which had compressibility of approximately 0.5 cc due to dissolved or entrapped air.

## Claims

1. A syringe, comprising:
a syringe body having a sidewall, and an opening at each of its proximal and distal ends;
a floating hydrophobic septum contacting the sidewall of the syringe body and
translatable from a position near the proximal end of the syringe body to a position at the distal end of the syringe body, thereby creating a top compartment, bounded by the proximal end of the syringe and the hydrophobic septum, and a bottom compartment, bounded by the distal end of the syringe and the hydrophobic septum,
a liquid in the top compartment, the liquid being an aqueous solution which comprises phosphates or borates;
a polymerizable component in the bottom compartment; and
a plunger having a shaft, a distal end, a proximal end, and a plunger head provided at the distal end; the plunger inserted into the syringe body plunger head first through the proximal opening of the syringe body; the plunger head configured and adapted to form a seal when the plunger head engages the sidewall of the syringe body, wherein the plunger is axially translatable between a position where the plunger head is at the proximal end of the syringe body and a position wherein the plunger head is flush with the hydrophobic septum, wherein the hydrophobic septum repels the liquid such that the liquid does not pass through the hydrophobic septum absent pressure applied by the plunger,
**characterized in that** the hydrophobic septum comprises a hydrophobic porous filter.

2. The syringe of claim 1, further comprising a cap, luer lock or frangible seal at the distal end of the syringe body.

3. The syringe of claim 1 or claim 2, wherein the hydrophobic septum is a hydrophobic porous filter.

4. The syringe of any preceding claim, wherein the liquid in the top compartment is buffer.

5. The syringe of any preceding claim, wherein the polymerizable material comprises a material selected from:
a polyalkyleneimine; and PEI.

6. The syringe of any preceding claim, further comprising a dissolution aid scaffold;
wherein the polymerizable component is adsorbed onto the dissolution aid scaffold, optionally wherein the dissolution aid scaffold is a piece of plastic or glass.

## Patentansprüche

1. Spritze, umfassend:
einen Spritzenkörper, der eine Seitenwand und eine Öffnung an jedem von seinem proximalen und seinem distalen Ende aufweist;
ein schwimmendes hydrophobes Septum, das die Seitenwand des Spritzenkörpers berührt und von einer Position nahe dem proximalen Ende des Spritzenkörpers in eine Position an dem distalen Ende des Spritzenkörpers verschiebbar ist, wodurch ein oberer Abschnitt, der durch das proximale Ende der Spritze und das hydrophobe Septum begrenzt wird, und ein unterer Abschnitt, der durch das distale Ende der Spritze und das hydrophobe Septum begrenzt wird, erzeugt werden,
eine Flüssigkeit in dem oberen Abschnitt, wobei die Flüssigkeit eine wässrige Lösung ist, die Phosphate oder Borate umfasst;
eine polymerisierbare Komponente in dem unteren Abschnitt; und
einen Kolben, der einen Schaft, ein distales Ende, ein proximales Ende und einen Kolbenkopf, der an dem distalen Ende bereitgestellt ist, aufweist; wobei der Kolben mit dem Kolbenkopf voran durch die proximale Öffnung des Spritzenkörpers in den Spritzenkörper eingeführt wird; wobei der Kolbenkopf so konfiguriert und ausgelegt ist, dass er eine Dichtung ausbildet, wenn der Kolbenkopf die Seitenwand des Spritzenkörpers in Eingriff nimmt, wobei der Kolben axial zwischen einer Position, in der sich der Kolbenkopf an dem proximalen Ende des Spritzenkörpers befindet, und einer Position, in der der Kolbenkopf bündig mit dem hydrophoben Septum ist, verschiebbar ist, wobei das hydrophobe Septum die Flüssigkeit so abstößt, dass die Flüssigkeit nicht durch das hydrophobe Septum gelangt, wenn kein Druck durch den Kolben aufgebracht wird,
**dadurch gekennzeichnet, dass** das hydrophobe Septum einen hydrophoben porösen Filter umfasst.

2. Spritze nach Anspruch 1, ferner eine Kappe, einen Luer-Lock oder eine zerbrechbare Dichtung an dem distalen Ende des Spritzenkörpers umfassend.

3. Spritze nach Anspruch 1 oder Anspruch 2, wobei das hydrophobe Septum ein hydrophober poröser Filter ist.

4. Spritze nach einem vorhergehenden Anspruch, wobei die Flüssigkeit in dem oberen Abschnitt ein Puffer ist.

5. Spritze nach einem vorhergehenden Anspruch, wobei das polymerisierbare Material ein Material umfasst, das aus Folgenden ausgewählt ist:
einem Polyalkylenimin; und PEI.

6. Spritze nach einem vorhergehenden Anspruch, ferner ein Auflösungshilfsgerüst umfassend; wobei die polymerisierbare Komponente auf dem Auflösungshilfsgerüst adsorbiert ist, optional wobei das Auflösungshilfsgerüst ein Stück Kunststoff oder Glas ist.

## Revendications

1. Seringue, comprenant :
un corps de seringue comportant une paroi latérale et une ouverture au niveau de chacune de ses extrémités proximale et distale ;
un septum hydrophobe flottant en contact avec la paroi latérale du corps de seringue et pouvant être translaté d'une position proche de l'extrémité proximale du corps de seringue à une position au niveau de l'extrémité distale du corps de seringue, créant ainsi un compartiment supérieur, délimité par l'extrémité proximale de la seringue et le septum hydrophobe, et un compartiment inférieur, délimité par l'extrémité distale de la seringue et le septum hydrophobe, un liquide dans le compartiment supérieur, le liquide étant une solution aqueuse qui comprend des phosphates ou des borates ;
un composant polymérisable dans le compartiment inférieur ; et
un piston comportant une tige, une extrémité distale, une extrémité proximale et une tête de piston disposée au niveau de l'extrémité distale ; le piston étant inséré dans la tête de piston du corps de seringue en premier à travers l'ouverture proximale du corps de seringue ; la tête de piston étant conçue et adaptée pour former un joint lorsque la tête de piston entre en prise avec la paroi latérale du corps de seringue, ledit piston pouvant être déplacé axialement entre une position dans laquelle la tête de piston se trouve au niveau de l'extrémité proximale du corps de seringue et une position dans laquelle la tête de piston affleure le septum hydrophobe, ledit septum hydrophobe repoussant le liquide de sorte que le liquide ne passe pas à travers le septum hydrophobe en l'absence de pression appliquée par le piston, **caractérisé en ce que** le septum hydrophobe comprend un filtre poreux hydrophobe.

2. Seringue selon la revendication 1, comprenant en outre un capuchon, un raccord luer ou un joint frangible au niveau de l'extrémité distale du corps de seringue.

3. Seringue selon la revendication 1 ou la revendication 2, ledit septum hydrophobe étant un filtre poreux hydrophobe.

4. Seringue selon l'une quelconque des revendications précédentes, ledit liquide dans le compartiment supérieur étant un tampon.

5. Seringue selon l'une quelconque des revendications précédentes, ledit matériau polymérisable comprenant un matériau choisi parmi :
une polyalkylèneimine ; et la PEI.

6. Seringue selon l'une quelconque des revendications précédentes, comprenant en outre un support d'aide à la dissolution ; ledit composant polymérisable étant adsorbé sur le support d'aide à la dissolution, éventuellement ledit support d'aide à la dissolution étant un morceau de plastique ou de verre.
